# EUROPEAN PATENT APPLICATION

(11) **EP 3 845 541 A1**
(43) Date of publication of application: **07.07.2021**
(21) Application number: 19856185.4
(22) Date of filing: 30.08.2019
(51) Int. Cl.: C07D 513/04, A61K 33/18, A61K 33/24, A61K 33/34, A61K 47/54, A61K 47/60, A61K 51/04, A61P 35/00, C07D 519/00, C07F 5/00

(54) **RADIOACTIVE IMIDAZOTHIADIAZOLE DERIVATIVE COMPOUND**

(30) Priority: 30.08.2018 JP 2018162085; 05.11.2018 JP 2018208322; 01.03.2019 JP 2019037734
(71) Applicant: Nihon Medi-Physics Co., Ltd, Koto-ku Tokyo, 136-0075 (JP); Kyoto University, Kyoto-shi, Kyoto 606-8501 (JP)
(72) Inventor: KIRIU Masato, Tokyo 136-0075 (JP); NAKATA Norihito, Tokyo 136-0075 (JP); ABE Tsutomu, Tokyo 136-0075 (JP); ONO Masahiro, Kyoto-shi, Kyoto 606-8501 (JP)
(74) Representative: TBK
(86) International application number: PCT/JP2019/034156
(87) International publication number: WO 2020/045638

(57) **Abstract**

The present invention provides a radiolabeled compound represented by the following formula (1), which is a radioactive imidazothiadiazole derivative compound having an affinity for CA-IX, or a salt thereof. 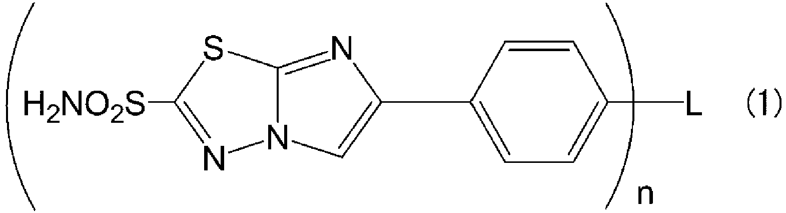 wherein n is an integer of 1 to 4, and L represents a radionuclide or a mono- to tetravalent group containing a radionuclide.

## Description

### Technical Field

The present invention relates to a radioactive imidazothiadiazole derivative compound having an affinity for carbonic anhydrase IX (hereinafter, also referred to as "CA-IX") and application of the same.

### Background Art

It is known that CA-IX is highly expressed in the hypoxic region of cancer, and it is expected that the diagnosis and treatment of cancer can be realized with the use of a compound targeting the CA-IX. For example, it has been reported that several compounds having a sulfonamide group at the terminal, such as acetazolamide, and 1,3,5-triazole benzene sulfonamide exhibit CA-IX inhibitory activity (Non Patent Literatures 1 and 2). Further, there is also an attempt to image the hypoxic region of cancer by labeling a compound that has an affinity for the CA-IX with a radionuclide (For example, Non Patent Literatures 3 and 4) .

The present applicants have filed a patent application for a radioactive metal complex targeting the CA-IX (Patent Literature 1).

Although the CA-IX is not targeted, there have been some reports that a compound having a sulfonamide group in the imidazothiadiazole skeleton inhibits a certain carbonic anhydrase like acetazolamide, and exhibits cerebral vasodilation (Non Patent Literature 5, and Patent Literature 2), and that the compound is useful for the prevention of neuronal cell loss or the treatment of neuronal or axonal degeneration (Patent Literature 3).

### Citation List

### Non Patent Literature

Non Patent Literature 1: V. Garaj et al, Bioorg. Med. Chem. Lett., 14, 2004, 5427-5433
Non Patent Literature 2: Ilies MA et al., J. Med. Chem. 2003 May 22; 46 (11): 2187-96
Non Patent Literature 3: Lau J, et al., Mol Pharm. 2016; 13: 1137-46
Non Patent Literature 4: Lv PC, et al., Bioconjugate Chem. 2016; 27: 1762-9
Non Patent Literature 5: Ian T. Barnish et al., J. Med. Chem. 1980, 23: 117-121

Patent Literature

Patent Literature 1: Japanese Patent Application No. 2017-169825
Patent Literature 2: U.K. Patent No. 1464259
Patent Literature 3: WO 2003/051890 A

### Summary of Invention

The present invention is to provide a radioactive imidazothiadiazole derivative compound having an affinity for CA-IX.

One embodiment of the present invention provides a radiolabeled compound represented by the following formula (1) or a salt thereof.

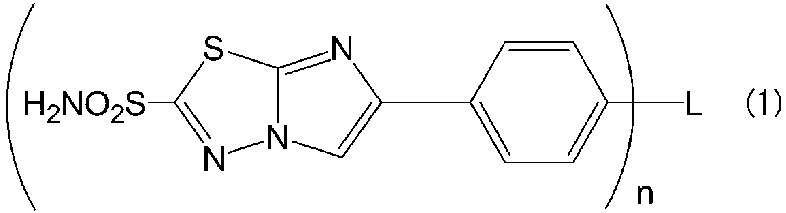

In the above formula (1), n is an integer of 1 to 4, and L represents a radionuclide or a mono- to tetravalent group containing a radionuclide.

Further, another embodiment of the present invention provides a radiopharmaceutical comprising the radiolabeled compound represented by the above formula (1) or a salt thereof, as an active component.

Furthermore, another embodiment of the present invention provides a compound represented by the following formula (2) or a salt thereof: 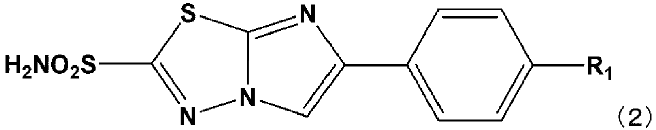 wherein R₁ represents a non-radioactive halogen atom, a nitro group, a trialkyl ammonium group, a dialkyl sulfonium group, a trialkyl stannyl group, a triphenyl stannyl group, a trialkyl silyl group, a triphenyl silyl group, an alkyl group having 1 to 10 carbon atoms which is replaced at a terminal thereof with a sulfonyloxy group, or a polyethylene glycol group having 1 to 10 carbon atoms which is replaced at a terminal thereof with a sulfonyloxy group.

Moreover, another embodiment of the present invention provides a method for producing a radioactive halogen-labeled compound represented by the following formula (3) or a salt thereof: 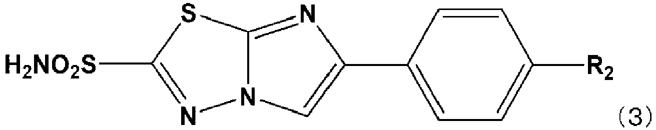 wherein R₂ represents a radioactive halogen atom, an alkyl group having 1 to 10 carbon atoms which is replaced at a terminal thereof with a radioactive halogen atom, or a polyethylene glycol group having 1 to 10 carbon atoms which is replaced at a terminal thereof with a radioactive halogen atom,
from the compound represented by the above formula (2) or a salt thereof, by a radioactive halogenation reaction.

In addition, another embodiment of the present invention provides a compound represented by the following formula (2-1), (2-2), (3-1), or (3-2), or a salt thereof: 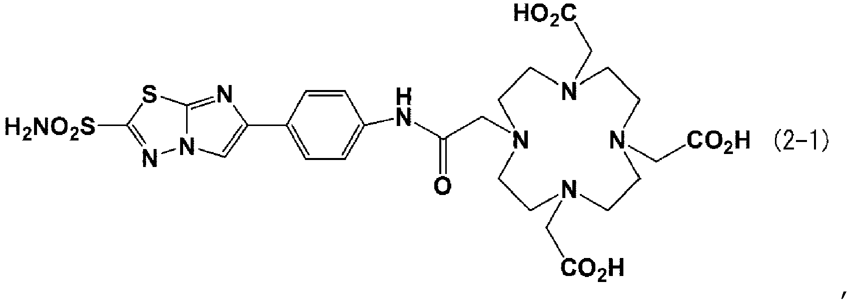 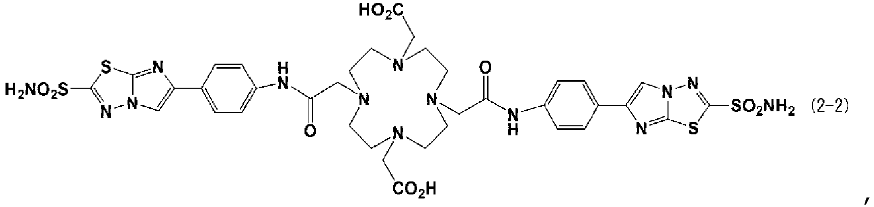 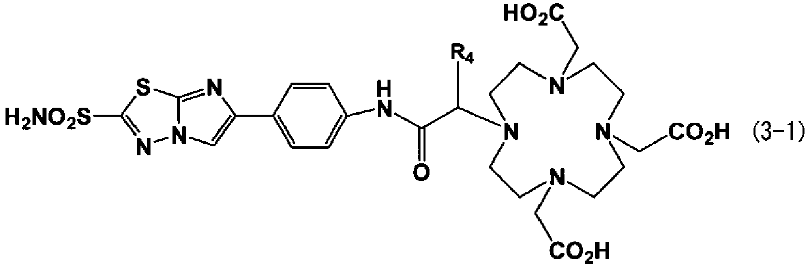 wherein R₄ represents H or CO₂H,
or

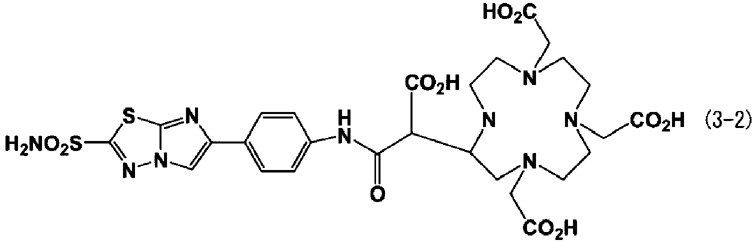

Further, another embodiment of the present invention provides a complex comprising the compound represented by the above formula (2-1), (2-2), (3-1), or (3-2), or a salt thereof with a radioactive metal.

Furthermore, another embodiment of the present invention provides a method for producing a radioactive metal complex, comprising mixing the compound represented by the above formula (2-1), (2-2), (3-1), or (3-2), or a salt thereof with a radioactive metal to obtain the above complex.

Moreover, another embodiment of the present invention provides a kit for preparing the above complex, comprising the compound represented by the above formula (2-1), (2-2), (3-1), or (3-2), or a salt thereof.

In addition, another embodiment of the present invention provide use of the radiolabeled compound represented by the above formula (1) or a salt thereof, or the compound represented by the above formula (2), (2-1), (2-2), (3-1), or (3-2), or a salt thereof, in production of a medicament.

According to the present invention, a radioactive imidazothiadiazole derivative compound having an affinity for CA-IX is provided.

### Brief Description of Drawings

Fig. 1 shows autoradiograms indicating the accumulation of radioactivity of [¹²³I]HIC205 obtained in Example 4 and the change in the accumulation in each treatment.
Fig. 2A is a view showing the comparison of the accumulation of radioactivity of [¹²³I]HIC205 measured in Example 4 with the CA-IX expression site.
Fig. 2B is an enlarged view showing the result of immunohistochemical staining of animal B shown in the lower left of Fig. 2A.
Fig. 3A is a diagram showing the results of Example 11.
Fig. 3B is a diagram showing the results of Example 11.
Fig. 3C is a diagram showing the results of Example 11.
Fig. 4A is a graph showing the change over time in the intact % of [¹¹¹In]ITDA1 obtained in Example 13.
Fig. 4B is a graph showing the change over time in the intact % of [¹¹¹In]ITDA2 obtained in Example 13.
Fig. 5 is a diagram showing the results of Example 15.
Fig. 6A is single-photon emission computed tomography/computed tomography (SPECT/CT) imaging results of a HT-29 cancer-bearing mouse obtained in Example 16, in which the arrows indicate the sites of tumor.
Fig. 6B is SPECT/CT imaging results of an MDA-MB-231 cancer-bearing mouse obtained in Example 16, in which the arrows indicate the sites of tumor.

### Description of Embodiments

In the present invention, the salt may be any salt that is acceptable as a medicament. For example, the salt may be a salt derived from an inorganic acid such as hydrochloric acid, hydrobromic acid, sulfuric acid, nitric acid, or phosphoric acid, or an organic acid such as acetic acid, trifluoroacetic acid, maleic acid, succinic acid, mandelic acid, fumaric acid, malonic acid, pyruvic acid, oxalic acid, glycolic acid, salicylic acid, a pyranosidyl acid (glucuronic acid, galacturonic acid, or the like), an α-hydroxy acid (citric acid, tartaric acid, or the like), an amino acid (aspartic acid, glutamic acid, or the like), an aromatic acid (benzoic acid, cinnamic acid, or the like), or a sulfonic acid (p-toluenesulfonic acid, ethanesulfonic acid, or the like).

In the present invention, the expression "radionuclide" refers to a nuclide having radioactivity, and preferably refers to a radioactive halogen atom, or a radioactive metal.

In the present invention, the expression "radioactive halogen atom" refers to at least one selected from radioactive isotopes of fluorine, chlorine, bromine, iodine, and astatine, and preferably ¹⁸F, ^{34m}Cl, ⁷⁶Br, ¹²³I, ¹²⁴I, ¹²⁵I, ¹³¹I, or ²¹¹At can be used as the radioactive halogen atom. In this regard, the expression "radioactive iodine atom" in the present invention refers to any one of ¹²³I, ¹²⁴I, ¹²⁵I, or ¹³¹I.

In the above formula (1), when n = 1, it is preferable that L represents a radioactive halogen atom, an alkyl group having 1 to 10 carbon atoms which is replaced at a terminal thereof with a radioactive halogen atom, or a polyethylene glycol group having 1 to 10 carbon atoms which is replaced at a terminal thereof with a radioactive halogen atom. The "alkyl group having 1 to 10 carbon atoms which is replaced at a terminal thereof with a radioactive halogen atom" can be represented by -(CH₂)ₘX (m is 1 to 10, and X represents a radioactive halogen atom). Further, the "polyethylene glycol group having 1 to 10 carbon atoms which is replaced at a terminal thereof with a radioactive halogen atom" can be represented by -(CH₂CH₂O)ₚX (p is 1 to 5, and X represents a radioactive halogen atom). When L represents a radioactive halogen atom, a radioactive iodine atom is preferable. In addition, when L represents an alkyl group having 1 to 10 carbon atoms which is replaced at a terminal thereof with a radioactive halogen atom, or a polyethylene glycol group having 1 to 10 carbon atoms which is replaced at a terminal thereof with a radioactive halogen atom, a radioactive fluorine atom is preferable as the radioactive halogen atom.

Among the compounds represented by the above formula (1) or salts thereof, the compound represented by the above formula (3) wherein R₂ represents a radioactive halogen atom, or a salt thereof can be produced from the compound represented by the above formula (2) wherein R₁ represents a non-radioactive halogen atom, a nitro group, a trialkyl ammonium group, a dialkyl sulfonium group, a trialkyl stannyl group, a triphenyl stannyl group, a trialkyl silyl group, or a triphenyl silyl group, or a salt thereof, by a radioactive halogenation reaction.

Among the compounds represented by the above formula (1) or salts thereof, the compound represented by the above formula (3) wherein R₂ represents an alkyl group having 1 to 10 carbon atoms which is replaced at a terminal thereof with a radioactive halogen atom, or a salt thereof can be produced from the compound represented by the above formula (2) wherein R₁ represents an alkyl group having 1 to 10 carbon atoms which is replaced at a terminal thereof with a sulfonyloxy group, or a salt thereof, by a radioactive halogenation reaction.

Among the compounds represented by the above formula (1) or salts thereof, the compound represented by the above formula (3) wherein R₂ represents a polyethylene glycol group having 1 to 10 carbon atoms which is replaced at a terminal thereof with a radioactive halogen atom, or a salt thereof can be produced from the compound represented by the above formula (2) wherein R₁ represents a polyethylene glycol group having 1 to 10 carbon atoms which is replaced at a terminal thereof with a sulfonyloxy group, or a salt thereof, by a radioactive halogenation reaction.

In the present invention, the non-radioactive halogen atom is at least one selected from a non-radioactive fluorine atom, a non-radioactive chlorine atom, a non-radioactive bromine atom, a non-radioactive iodine atom, and a non-radioactive astatine atom.

In the present invention, an example of the trialkyl ammonium group includes a tri(C1-C4 alkyl) ammonium group, and more preferably a trimethylammonium group. Further, a preferable example of the dialkyl sulfonium group includes a di(C1-C4 alkyl) sulfonium group. Furthermore, an example of the trialkyl stannyl group includes a tri(C1-C4 alkyl) tin group, and more preferably a tributyl stannyl group. Moreover, an example of the trialkyl silyl group includes a tri(C1-C4 alkyl) silyl group, and more preferably a trimethyl silyl group. In addition, the alkyl group having 1 to 10 carbon atoms which is replaced at a terminal thereof with a sulfonyloxy group can be represented by-(CH₂)ₘR₃ wherein m is 1 to 10, and R₃ represents a sulfonyloxy group. Further, the polyethylene glycol group which is replaced at a terminal thereof with a sulfonyloxy group can be represented by -(CH₂CH₂O)ₚR₃ wherein p is 1 to 5, and R₃ represents a sulfonyloxy group.

In this regard, in the present invention, the expression "C1-C4 alkyl" means a linear or branched alkyl group having 1 to 4 carbon atoms. Further, examples of the sulfonyloxy group include a linear or branched alkylsulfonyloxy group having 1 to 10 carbon atoms (for example, mesylate group), a linear or branched halogenoalkylsulfonyloxy group having 1 to 10 carbon atoms (for example, triflate group), and a substituted or unsubstituted arylsulfonyloxy group (for example, tosylate group) .

The compound represented by the above formula (2) wherein R₁ represents a trialkyl stannyl group, a triphenyl stannyl group, a trialkyl silyl group, or a triphenyl silyl group, or a salt thereof can undergo the radioactive halogenation reaction by way of an electrophilic substitution reaction. In addition, the compound represented by the above formula (2) wherein R₁ represents a nitro group, a trialkyl ammonium group, a dialkyl sulfonium group, an alkyl group having 1 to 10 carbon atoms which is replaced at a terminal thereof with a sulfonyloxy group, or a polyethylene glycol group having 1 to 10 carbon atoms which is replaced at a terminal thereof with a sulfonyloxy group, or a salt thereof can undergo the radioactive halogenation reaction by way of a nucleophilic substitution reaction.

In a case where the radioactive halogenation reaction is performed by an electrophilic substitution reaction, it may be performed by using a radioactive halogen prepared as an electrophile, and can be performed by using, for example, a radioactive halogen molecule or a radioactive acetyl hypohalite. Examples of the radioactive halogen molecule include a radioactive fluorine molecule, a radioactive chlorine molecule, a radioactive bromine molecule, a radioactive iodine molecule, and a radioactive astatine molecule. Examples of the radioactive acetyl hypohalite include radioactive acetyl hypofluorite, radioactive acetyl hypochlorite, radioactive acetyl hypobromite, and radioactive acetyl hypoiodite. Alternatively, the reaction may be performed by reaction with a radioactive sodium halide or a radioactive potassium halide in the presence of an oxidizing agent under an acidic condition. As the oxidizing agent, for example, chloramine-T, hydrogen peroxide water, peracetic acid, halogenated succinimide, or the like can be used.

Further, in a case where the radioactive halogenation reaction is performed by a nucleophilic substitution reaction, the radioactive halogenation reaction may be performed by using a radioactive halogen prepared as a nucleophile, and can be performed by using, for example, radioactive halide ions. Examples of the radioactive halide ions include radioactive fluoride ions, radioactive chloride ions, radioactive bromide ions, radioactive iodide ions, and radioactive astatine ions. Moreover, the reaction may be performed in the presence of a base.

For example, by performing a radioactive iodination reaction with the compound represented by the above formula (2) wherein R₁ represents a non-radioactive iodine atom, a trialkyl stannyl group, a triphenyl stannyl group, a trialkylsilyl group, or a triphenyl stannyl group by using a radioactive alkali metal iodide, a compound represented by the above formula (1) wherein L represents a radioactive iodine atom (compound represented by the above formula (3) wherein R₂ represents a radioactive iodine atom) can be obtained. It is preferable to perform the radioactive iodination reaction by reaction with a radioactive alkali metal iodide and an oxidizing agent under an acidic condition. As the radioactive alkali metal iodide, for example, a sodium compound with radioactive iodine, or a potassium compound with radioactive iodine can be used. As the oxidizing agent, for example, chloramine-T, hydrogen peroxide water, peracetic acid, N-chlorosuccinimide, N-bromosuccinimide, or the like can be used. As an example, by performing a radioactive iodination reaction by reaction with radioactive sodium iodide (for example, [¹²³I] sodium iodide, [¹²⁴I] sodium iodide, [¹²⁵I] sodium iodide, or [¹³¹I] sodium iodide), in the presence of an oxidizing agent such as hydrogen peroxide water under an acidic condition such as hydrochloric acid, a radiolabeled compound represented by the above formula (1) wherein L represents a radioactive iodine atom and n = 1 can be obtained.

Further, by performing a radioactive fluorination reaction with the compound represented by the above formula (2) wherein R₁ represents an alkyl group having 1 to 10 carbon atoms which is replaced at a terminal thereof with a sulfonyloxy group by using radioactive fluoride ions, a compound represented by the above formula (3) wherein R₂ represents an alkyl group having 1 to 10 carbon atoms which is replaced at a terminal thereof with a radioactive fluorine atom can be obtained.

Further, by performing a radioactive fluorination reaction with the compound represented by the above formula (2) wherein R₁ represents a polyethylene glycol group having 1 to 10 carbon atoms which is replaced at a terminal thereof with a sulfonyloxy group by using radioactive fluoride ions, a compound represented by the above formula (3) wherein R₂ represents a polyethylene glycol group having 1 to 10 carbon atoms which is replaced at a terminal thereof with a radioactive fluorine atom can be obtained.

In addition, by performing a radioactive fluorination reaction with the compound represented by the above formula (2) wherein R₁ represents a dialkyl sulfonium group by using radioactive fluoride ions, a compound represented by the above formula (3) wherein R₂ represents a radioactive fluorine atom can be obtained.

In this regard, in a case where the radioactive fluorination reaction is performed, the reaction can also be performed in the presence of potassium carbonate and a cryptand, or in the presence of tetrabutylammonium hydrogen carbonate.

In the present invention, in a case where L represents a group containing a radionuclide in the compound represented by the above formula (1), the group is preferably a mono- to tetravalent ligand carrying a radioactive metal, and in this case, when n is 1 or 2 in the above formula (1), the water solubility becomes lower, and thus, the compound is more preferable as a radiopharmaceutical. Examples of the ligand include ethylenediamine tetraacetic acid (EDTA), diethylenetriamine pentaacetic acid (DTPA), triethylenetetramine hexaacetic acid (TTHA), cyclam, 1,4,8,11-tetraazacyclotetradecane-1,4,8,11-tetraacetic acid (TETA), 1,4,7,10-tetraazacyclododecane-1,4,7,10-tetraacetic acid (DOTA), N{1-2,3-dioleyloxy}propyl}-N,N,N,-triethylammonium (DOTMA), mercaptoacetylglycylglycine (MAG3), ethylene cysteine dimer (ECD), hydrazinonicotinyl (HYNIC), lysine-tyrosine-cysteine (KYC), cysteine-glycine-cysteine (CYC), N,N'-bis(mercaptoacetamide)ethylenediamine (DADS), N,N'-bis(mercaptoacetamide)-2,3 diamine propanoic acid (CO2DADS), N,N'-bis(2-mercaptoethyl)ethylenediamine (BATs), thiosemicarbazone, propylene amine oxime (PnAO), and other amine oxime ligands and derivatives thereof. Among them, from the viewpoint of increasing the accumulation in a tumor, DOTA in which at least one carboxy terminus is amidated is preferable. Further, from the viewpoint of reducing the accumulation in normal tissues, DOTA in which four carboxyl groups are intact, or a derivative thereof is preferable, and for example, DOTAGA is preferable.

In the present invention, the radioactive metal is not particularly limited as long as it forms a chelate with the above ligand, and examples of the radioactive metal include radioactive isotopes of indium, gallium, actinium, yttrium, lutetium, technetium, copper, gadolinium, and bismuth (specifically, ¹¹¹In, ⁹⁰Y, ⁶⁸Ga, ¹⁷⁷Lu, ^{99m}Tc, ⁶⁴Cu, ¹⁵³Gd, ²¹³Bi, and ²²⁵Ac). In a case where a radiolabeled compound represented by the above formula (1), or a complex of the compound represented by the above formula (2-1), (2-2), (3-1), or (3-2), or a salt thereof with a radioactive metal, is used as an imaging agent, a radioactive metal that emits positrons or gamma rays is used. Examples of the radioactive metal that emits gamma rays include ¹¹¹In, ⁶⁷Ga, ⁶⁸Ga, ^{99m}Tc, ⁶⁴Cu, and ¹⁵³Gd. In a case where a radiolabeled compound represented by the above formula (1), or a complex of the compound represented by the above formula (2-1), (2-2), (3-1), or (3-2), or a salt thereof with a radioactive metal, is used as an internal radiotherapeutic agent, a radioactive metal that emits alpha rays or beta rays is used. Examples of the radioactive metal that emits alpha rays or beta rays include ²²⁵Ac, ⁹⁰Y, ¹⁷⁷Lu, ⁶⁴Cu, and ²¹³Bi.

The complex of the compound represented by the above formula (2-1), (2-2), (3-1), or (3-2), or a salt thereof with a radioactive metal can be produced by mixing the compound represented by the above formula (2-1), (2-2), (3-1), or (3-2), or a salt thereof with a radioactive metal as they are or in the presence of a solvent. The complex may be prepared by using a kit comprising the compound represented by the above formula (2-1), (2-2), (3-1), or (3-2), or a salt thereof. The kit comprises the compound represented by the above formula (2-1), (2-2), (3-1), or (3-2), or a salt thereof as it is or in a state of being dissolved in a solvent. In a case where the kit comprises the compound represented by the above formula (2-1), (2-2), (3-1), or (3-2), or a salt thereof as it is, the compound represented by the above formula (2-1), (2-2), (3-1), or (3-2), or a salt thereof can be in a powder form, , for example, freeze-dried powder.

Among the radiolabeled compounds represented by the above formula (1), a compound in which L represents DOTA, and n is 1 or 2 is preferable.

Therefore, according to the preferable embodiment of the present invention, a radiolabeled compound represented by the following formula (1-1) or (1-2) is provided. 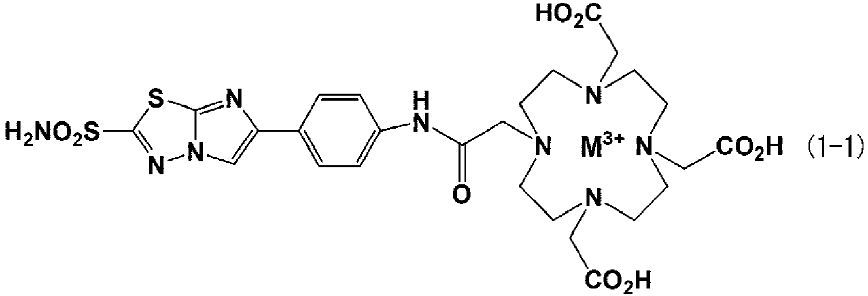 wherein M³⁺ represents a trivalent radioactive metal element. 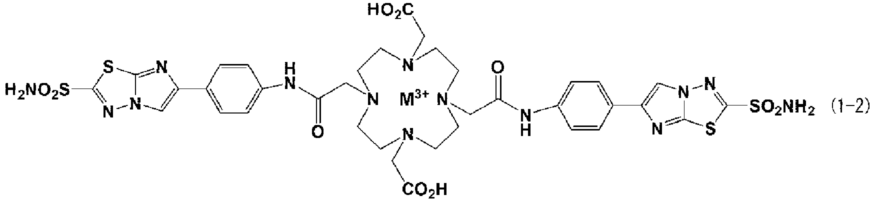 wherein M³⁺ represents a trivalent radioactive metal element.

Further, from the viewpoint of reducing the accumulation in normal tissues, a radiolabeled compound represented by the above formula (1) wherein L represents a ligand represented by the following formula (1-3) or (1-4) in which a radioactive metal is carried, and n is 1 is preferable. 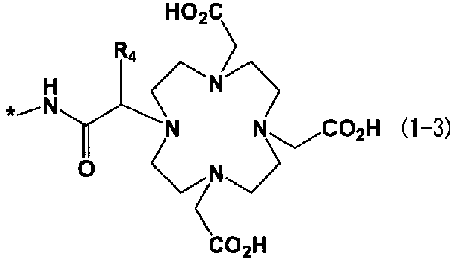 wherein R₄ represents H or CO₂H, and a pentagram asterisk represents a binding site. 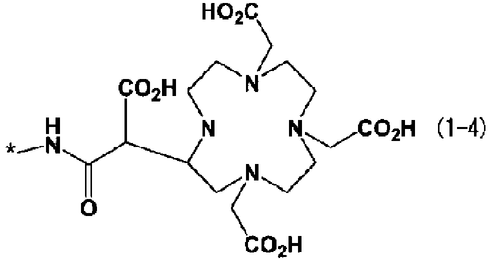 wherein a pentagram asterisk represents a binding site.

The trivalent radioactive metal element represented by M³⁺ in the radiolabeled compound represented by the above formula (1-1) or (1-2), or a salt thereof is not particularly limited as long as it forms a chelate with the above ligand, and examples of the trivalent radioactive metal element include indium, gallium, actinium, yttrium, and lutetium. In a case where the radiolabeled compound represented by the above formula (1-1) or (1-2), or a salt thereof is used as an imaging agent, a trivalent radioactive metal element that emits positrons or gamma rays is used as the M³⁺. Examples of the trivalent radioactive metal element that emits positrons or gamma rays include ¹¹¹In, ⁶⁷Ga, and ⁶⁸Ga. In a case where the radiolabeled compound represented by the above formula (1-1) or (1-2), or a salt thereof is used as an internal radiotherapeutic agent, a trivalent radioactive metal element that emits alpha rays or beta-minus rays is used as the M³⁺. Examples of the trivalent radioactive metal element that emits alpha rays or beta-minus rays include ²²⁵Ac, ⁹⁰Y, and ¹⁷⁷Lu.

The radiolabeled compound represented by the above formula (1-1) or (1-2), or a salt thereof can be produced by incubating the compound represented by the above formula (2-1) or (2-2), or a salt thereof and a metal ion M³⁺ as they are or in a solvent. As the metal ion M³⁺, a salt such as a chloride of metal M³⁺ can be used. The temperature and time for the incubation vary depending on the type of the metal ion M³⁺, and preferable conditions may be employed depending on the type of the metal ion M³⁺ to be used.

For example, the compound represented by the above formula (2-1) or (2-2), or a salt thereof and the chloride of metal M³⁺ are incubated in a solvent under a weakly acidic condition with pH 4 to 6. As the solvent, a pH buffer solution can be used, and examples of the pH buffer solution include a MES buffer solution, and a sodium acetate buffer solution. The incubation may be performed under heating or microwave irradiation. The obtained compounds represented by the above formula (1-1) and (1-2), or salts thereof can be purified by high performance liquid chromatography (HPLC) or the like.

The compound represented by the above formula (1) contains a sulfonamide group that is a CA-IX ligand, at the 2-position of 6-phenylimidazo[2,1-b][1,3,4]thiadiazole, and therefore, is considered to have a physiological activity of specifically accumulating in the CA-IX. Examples of the tumor expressing CA-IX include solid tumors in cancers of bladder, uterine cervix, head and neck, breast, lungs, kidney, and the like, and CA-IX is specifically expressed in a cancer cell membrane in the hypoxic region of a solid tumor. Accordingly, in another embodiment of the present invention, the radiolabeled compound represented by the above formula (1) can be made into a radiopharmaceutical that targets CA-IX, by prescribing the radiolabeled compound in a form suitable for administration into a living body.

It is preferable that the radiopharmaceutical according to the present invention is parenterally administered, and a more preferable dosage form thereof is an injection. Preferably, the radiopharmaceutical is in a form of aqueous solution, and may appropriately contain an additional component such as a pH regulator, a pharmaceutically acceptable solubilizer, an isotonizing agent, a stabilizer, or an antioxidant.

In a case where the radionuclide is a radionuclide that emits positrons or gamma rays, the radiolabeled compound represented by the above formula (1) specifically accumulates at the CA-IX expression site of a tumor, and thus the site can be visualized by nuclear medicine examination. Accordingly, the radiopharmaceutical according to the present invention can be used as an imaging agent for a tumor expressing the CA-IX.

In the present invention, the expression "imaging agent" means a radiopharmaceutical to be administered into the body for the purpose of nuclear medicine examination.

In the present invention, the content of the compound represented by the above formula (1) in the imaging agent is not particularly limited as long as the imaging agent at the time of use has an amount of radioactivity enough for the nuclear medicine examination, and if the amount of radioactivity is, for example, 50 to 740 MBq at the time of use, it is practical for use in the imaging for an adult.

In the present invention, examples of the nuclear medicine examination include positron emission tomography (PET), and single-photon emission computed tomography (SPECT), and specifically, the nuclear medicine examination means examination that enables non-invasive diagnosis of a pathological condition by administering an imaging agent into a living body and then detecting and imaging the radiation emitted from the body with a PET device, a SPECT device, or the like. In a case where a radionuclide that emits positrons is used as the radionuclide of the compound represented by the above formula (1), PET is suitable, and in a case where a radionuclide that emits gamma rays is used as the radionuclide, SPECT is suitable.

The method for imaging a tumor by using the radiolabeled compound represented by the above formula (1) may include a step of forming image data that include a visualized image of the CA-IX expression site of a tumor of an administered subject by performing tomography of the subject by nuclear medicine examination. In this regard, the "subject" means a living body that is subjected to nuclear medicine examination, and includes humans and animals.

Further, in a case where the radionuclide is a radionuclide that emits alpha rays or beta-minus rays, the radiolabeled compound represented by the above formula (1) specifically accumulates at the CA-IX expression site in the hypoxic region of a tumor such that the radiation therapy can be performed on the site. Accordingly, the radiopharmaceutical according to the present invention can be used as an internal radiotherapeutic agent for a tumor expressing the CA-IX.

In the present invention, the content of the compound represented by the above formula (1) in the internal radiotherapeutic agent is not particularly limited as long as the internal radiotherapeutic agent at the time of use has an amount of radioactivity enough for the internal radiation therapy.

The internal radiation therapy method of a tumor with the use of the radiolabeled compound represented by the above formula (1) may include a step of administering the radiolabeled compound to a patient with a tumor expressing the CA-IX. In this regard, the "patient" means a living body that is subjected to the internal radiation therapy, and includes humans and animals.

In addition, by combining the radiolabeled compound represented by the above formula (1) which radionuclide is a radionuclide that emits alpha rays or beta-minus rays or a salt thereof with the radiolabeled compound represented by the above formula (1) which radionuclide is a radionuclide that emits positrons or gamma rays or a salt thereof, the theranostics of a tumor expressing the CA-IX can be realized. Therefore, the radiopharmaceutical according to the present invention can also be used as a medicament for theranostics of a tumor expressing the CA-IX, which contains the radiolabeled compound represented by the above formula (1) which radionuclide is a radionuclide that emits alpha rays or beta-minus rays and the radiolabeled compound represented by the above formula (1) which radionuclide is a radionuclide that emits gamma rays, both as the active components.

The radiolabeled compound represented by the above formula (1) according to the present invention is useful for the internal radiation therapy of cancer and can further be applied to the theranostics of cancer since the radiolabeled compound specifically accumulates at the site of a tumor expressing the CA-IX and further shows favorable clearance when administered to a living body, such that not only the site of a tumor expressing the CA-IX can be more clearly imaged by nuclear medicine examination, but also the growth of a tumor expressing the CA-IX can be suppressed, and further the suppression of treatment resistance to chemotherapy or radiation therapy can be expected.

### Examples

Hereinafter, the present invention will be described in more detail by way of Examples, but the present invention is not limited to the contents of the following Examples.

In Examples, the molecular structure of each compound was identified by NMR spectrum (NMR device used: JNM-ECP-500 (manufactured by JEOL Ltd., used in Examples 1 and 2), or JNM-ECS-400 (manufactured by JEOL Ltd., used in Examples 6 to 8 and 10). All of the chemical shifts are in ppm on the delta-scale (δ), and the fine splittings of the signals are indicated by using abbreviations (s: singlet, d: doublet, m: multiplet, and br: broad). For the atmospheric pressure chemical ionization mass spectrometry (APCI-MS), the measurement was performed by using High Performance Liquid Chromatograph Mass Spectrometer LCMS-2020 manufactured by Shimadzu Corporation.

Hereinafter, the "room temperature" indicates 25°C in the following Examples.

### Example 1: Synthesis of labeling precursor compound

As a labeling precursor compound, 6-(4-bromophenyl)imidazo[2,1-b] [1,3,4]thiadiazole-2-sulfonamide) (compound 3) was prepared in accordance with the following scheme.

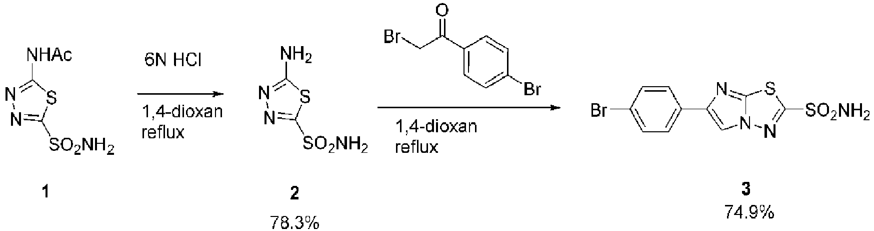

### (1) Synthesis of 5-amino-1,3,4-thiadiazole-2-sulfonamide (compound 2)

5.0 g (corresponding to 22.5 mmol) of acetazolamide (compound 1) was dissolved in 20 mL of methanol, 10 mL of 6 mol/mL hydrochloric acid was added to the obtained solution, and the obtained mixture was heated under reflux for 63.5 hours. After completion of the reaction, the reaction mixture was concentrated, and the neutralization was performed by adding a saturated sodium bicarbonate solution to the residue. The solid components were filtered, and then extracted with ethyl acetate (100 mL) three times. The combined ethyl acetate layer was concentrated, and the obtained crude product was purified by silica gel column chromatography (eluent: chloroform/methanol = 5/1) to obtain 3.18 g (corresponding to 17.6 mmol) of compound 2.

¹H-NMR (solvent: deuterated dimethyl sulfoxide, resonance frequency: 500 MHz): δ 8.06-8.05 (br, 2H), 7.84 (br, 2H).

### (2) Synthesis of 6-(4-bromophenyl)imidazo[2,1-b][1,3,4]thiadiazole-2-sulfonamide (compound 3)

1.4 g (corresponding to 5.04 mmol) of 2,4'-dibromoacetophenone and 1.0 g (corresponding to 5.6 mmol) of 5-amino-1,3,4-thiadiazole-2-sulfonamide (compound 2) were dissolved in 12.5 mL of 1,4-dioxane, and the obtained solution was stirred for 17 hours under heating reflux. After completion of the reaction, the reaction mixture was ice-cooled, and the precipitated solid was collected by filtration. The obtained crude product was dissolved in ethyl acetate/methanol = 5/1, hexane was added to the obtained solution to perform reprecipitation. The precipitated solid was collected by filtration, and dried under vacuum to obtain 1.36 g (corresponding to 3.77 mmol) of compound 3.

¹H-NMR (solvent: deuterated dimethyl sulfoxide, resonance frequency: 500 MHz): δ 8.94-8.93 (m, 1H), 8.73 (br, 2H), 7.88-7.84 (m, 2H), 7.68-7.63 (m, 2H).

### Example 2: Synthesis of 6-(4-(iodophenyl)imidazo[2,1-b][1,3,4]thiadiazole-2-sulfonamide (non-radioactive HIC205)

Non-radioactive HIC205 was prepared in accordance with the following scheme.

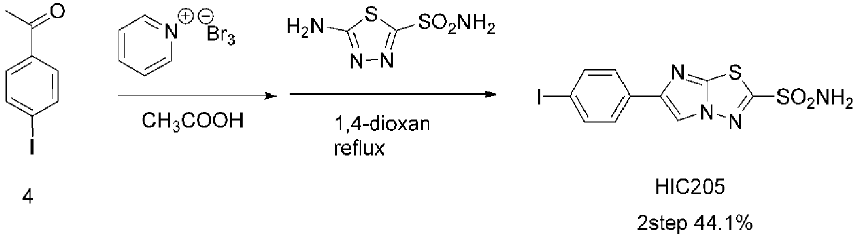

246 mg (corresponding to 1.0 mmol) of 4'-iodoacetophenone was dissolved in 3.0 mL of acetic acid, 384 mg (corresponding to 1.2 mmol) of pyridinium tribromide was added to the obtained solution, and the obtained mixture was stirred at room temperature for one hour. After completion of the reaction, the solvent was concentrated, and dried overnight under vacuum. To the obtained solid matter, 180 mg of 5-amino-1,3,4-thiadiazole-2-sulfonamide and 2.5 mL of 1,4-dioxane were added, and the obtained mixture was stirred for 20 hours under heating reflux. After completion of the reaction, the solvent was removed, and then 5 mL of water was added to the residue, and the precipitated solid was collected by filtration. The obtained crude product was dissolved in ethyl acetate/methanol = 5/1,, hexane was added to the obtained solution to perform reprecipitation. The precipitated solid was collected by filtration, and dried under vacuum to obtain 179 mg (corresponding to 0.441 mmol) of 6-(4-iodophenyl)imidazo[2,1-b] [1,3,4]thiadiazole-2-sulfonamide (HIC205).

¹H-NMR (solvent: deuterated dimethyl sulfoxide, resonance frequency: 500 MHz): δ 8.95-8.93 (m, 1H), 8.73 (br, 2H), 7.82-7.78 (m, 2H), 7.72-7.68 (m, 2H).

### Example 3: Synthesis of 6-(4-[¹²³I](iodophenyl)imidazo[2,1-b][1,3,4]thiadiazole-2-sulfonamide([¹²³I]HIC205)

In accordance with the following scheme, a ¹²³I-labeled compound [¹²³I]HIC205 was synthesized from the labeling precursor compound (compound 3) obtained in Example 1.

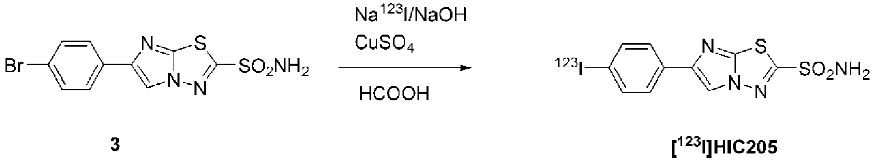

10 mg of copper sulfate pentahydrate was dissolved in 2.0 mL of formic acid to prepare a 2 µmol/mL copper sulfate formic acid solution. 1 mg of a labeling precursor compound (compound 3) was dissolved in 100 µL of the above-prepared 2 µmol/mL copper sulfate formic acid solution, and then into the obtained solution, 40 µL of [¹²³I] sodium iodide-containing 1 mol/L sodium hydroxide solution (amount of radioactivity: 679 MBq, correction value at the start of synthesis) was added, and the obtained mixture was heated at 132°C for one hour. After completion of the reaction, the resultant mixture was cooled to room temperature, and then into the cooled mixture, 200 µL of water for injection was added, and a [¹²³I]HIC205 fraction was collected by HPLC under the following conditions. In this regard, it was confirmed that the retention time of the [¹²³I]HIC205 fraction was the same as that of the non-radioactive HIC205.

### <HPLC conditions>

Column: COSMOSIL (trade name, manufactured by NACALAI TESQUE, INC., size: 4.6 mmΦ × 250 mm)
Mobile phase A: 0.1% aqueous solution of trifluoroacetic acid
Mobile phase B: 0.1% trifluoroacetic acid in acetonitrile
Gradient: 0 minutes (mobile phase A/mobile phase B = 40/60 (volume ratio)), and 120 minutes (80/20 (volume ratio))
Flow rate: 1.0 mL/min
Detector: ultraviolet-visible absorptiometer (detection wavelength: 254 nm), and radiation detector

2.0 mL of ethanol was added to the fraction, the obtained solution was heated at 90°C to distill off the solvent until the solvent became around 0.5 mL. After that, a [¹²³I]HIC205 fraction was collected again by HPLC under the above conditions. The fractionation liquid was passed through Sep-Pak tC2 Plus (trade name, manufactured by Waters Corporation Japan) to adsorb and capture [¹²³I]HIC205 on the column. The column was washed with 1 mL of water, and then 1 mL of diethyl ether was passed through the column to elute [¹²³I]HIC205. The amount of radioactivity obtained was 11.8 MBq (202 minutes after the start of synthesis). Further, as a result of thin-layer chromatography (TLC) analysis under the following conditions, the radiochemical purity was 98%.

### <TLC analysis conditions>

TLC plate: Silica Gel 60 F254 (product name, manufactured by Merck KGaA)
Development phase: hexane/ethyl acetate = 1/1
Detector: Gina Star (product name, manufactured by Raytest GmbH)

### Example 4: Examination of affinity of non-radioactive HIC205 for CA-IX

The affinity was measured by using Biacore (manufactured by GE Healthcare). Human CA-IX (manufactured by R&D Systems Inc.) was immobilized on a sensor chip (CM7) by an amino coupling method. After non-radioactive HIC205 was dissolved in 100% dimethyl sulfoxide (DMSO), the obtained solution was diluted with a running buffer in a stepwise manner by 10 times, and a non-radioactive HIC205 solution containing 0.1% DMSO was finally prepared. Based on this, a 2-fold dilution series was prepared with the running buffer containing 0.1% DMSO, and the measurement was performed. In this regard, the composition of the running buffer was 0.01 M HEPES (pH 7.4), 0.15M sodium chloride, and 0.005% v/v Surfactant P20. The sensorgram obtained from each concentration was directly curve fitted by a non-linear least-squares method, and the affinity was calculated. The results are shown in Table 1.

**Table 1**

| R | Affinity | Binding constant (×10⁵) [1/M·S] | Dissociation constant (×10⁻³) [1/s] |
|---|---|---|---|
| I (n = 1) | 0.109 nM | 83.1 | 0.903 |

### Example 5: In vitro autoradiography using [¹²³I]HIC205

Fresh-frozen sections (each 14 µm) were prepared from a tumor having a tumor volume of around 200 mm³, and then the prepared fresh-frozen sections were sufficiently air dried. The dried fresh-frozen sections were immersed in Tris-buffered saline (TBS), and then immersed in a TBS adjusted so as to have 5 kBq/ml of [¹²³I]HIC205 and a solution containing 400 µM acetazolamide in the TBS (both contained 0.1% dimethyl sulfoxide (DMSO)), and both the sections were incubated at the same time (at 37°C for 30 minutes). After that, the resultant sections were washed with TBS and 40% ethanol (EtOH)-containing TBS, respectively, and then air dried. The air-dried sections were each brought into contact with an imaging plate (IP), and were read out by a bioimaging analyzer BAS2000 (manufactured by FUJIFILM Corporation). The section used for autoradiography (ARG) was directly subjected to immunohistochemistry that was performed by using a goat-derived anti-CA-IX antibody.

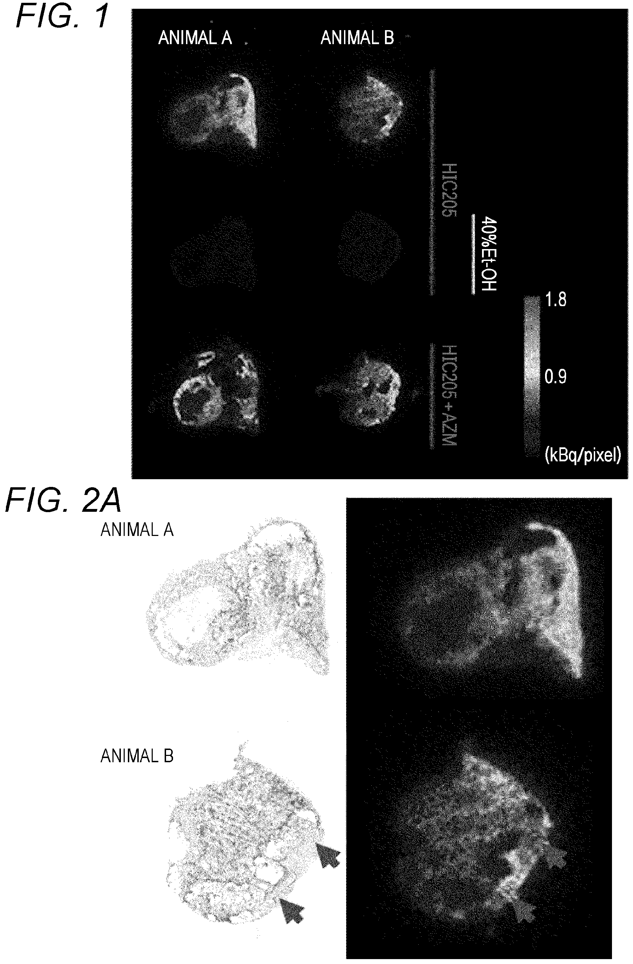

The localization of the radioactivity of [¹²³I]HIC205 and the difference in the binding of [¹²³I]HIC205 between the individual treatments are shown Fig. 1. The upper part shows the results obtained by incubating the tumor section in [¹²³I]HIC205-containing Tris-buffered saline, and then washing the incubated section with TBS, the middle part shows the results obtained in a similar manner as in the case of the upper part except that the section was washed with 40% ethanol-containing TBS in place of the TBS, and the lower part shows the results obtained in a similar manner as in the case of the upper part except that the tumor section was incubated in [¹²³I]HIC205-containing Tris-buffered saline and an acetazolamide-containing TBS solution at the same time.

From Fig. 1, the binding of [¹²³I]HIC205 was confirmed with contrast on the tumor section. Further, it was confirmed that the binding of [¹²³I]HIC205 was decreased by the simultaneous incubation with acetazolamide. In addition, by washing the tumor section with 40% ethanol, the localization of the radioactivity was significantly reduced.

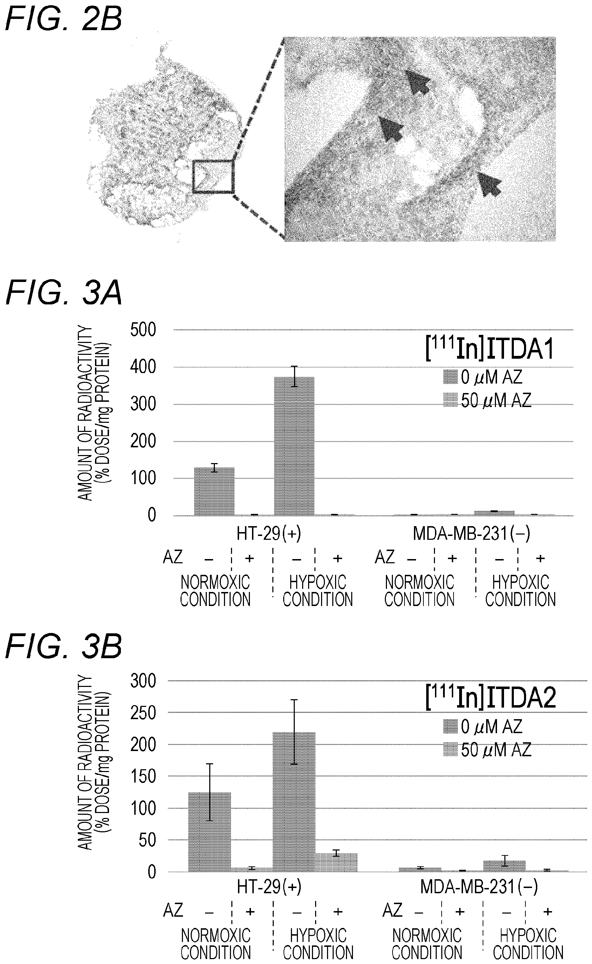

The results of the comparison of the localization of the radioactivity of [¹²³I]HIC205 with the expression of CA-IX are shown in Figs. 2A and 2B. Fig. 2A shows the results of autoradiograms of tumor sections on the right side, and the results of immunohistochemical staining of tumor sections on the left side. The region between two arrows indicates the part where the binding of [¹²³I]HIC205 and the expression of CA-IX are not consistent. Further, Fig. 2B is an enlarged view showing the region between the two arrows of the result of immunohistochemical staining of animal B shown in the lower left of Fig. 2A, in which the result of immunohistochemical staining of Fig. 2A was shown on the left side, and an enlarged view of the region between the two arrows was shown on the right side. The parts indicated by the arrows indicate the overlap of the section.

From the results of two examples in Fig. 2A, it was confirmed that the localization of [¹²³I]HIC205 and the expression site of CA-IX were mostly consistent. Even though some binding of [¹²³I]HIC205 was confirmed at the site where CA-IX was not expressed, the result of observation in a high-power field confirmed that the site was where the section overlapped (Fig. 2B).

From the results described above, it was confirmed that [¹²³I]HIC205 has binding ability to CA-IX.

### Example 6: Synthesis of targeting site

A targeting site was prepared in accordance with the following scheme.

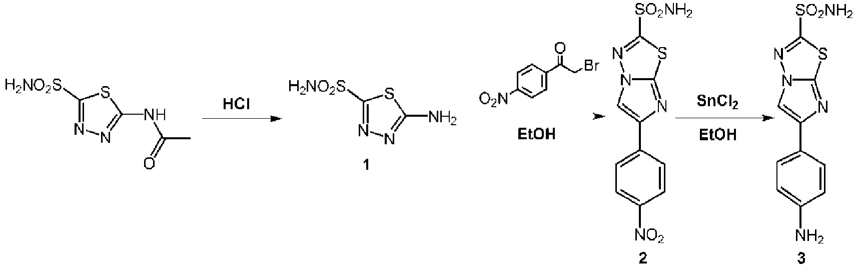

### (1) Synthesis of 5-amino-1,3,4-thiadiazole-2-sulfonamide (compound 1)

N-(5-sulfamoyl-1,3,4-thiadiazol-2-yl)acetamide (acetazolamide) (3,000 mg, 13.5 mmol) was suspended in 3 N hydrochloric acid aqueous solution (25 mL), and the obtained suspension was refluxed at 110°C for 2 hours. The resultant mixture was neutralized with NaHCO₃, and then the neutralized mixture was extracted with ethyl acetate (200 mL × 3). The organic layer was recovered, the solvent was distilled off, and then the residue was dried under vacuum to obtain the target product 1 as white powder (yield: 1,574 mg, 64%).
¹H-NMR (400 MHz, DMSO-d₆) δ 8.07 (s, 2H), 7.83 (s, 2H)
¹³C-NMR (100 MHz, DMSO-d₆) δ 171.6, 157.9
MS(ESI): m/z, C₂H₃N₄O₂S₂⁻, calculation value: 179.0, and measurement value: 179.0 ([M-H]⁻).

### (2) Synthesis of 6-(4-nitrophenyl)imidazo[2,1-b][1,3,4]thiadiazole-2-sulfonamide (compound 2)

A compound 2 (1,574 mg, 8.73 mmol) and 2-bromo-1-(4-nitrophenyl)ethane-1-one (2,131 mg, 8.73 mmol) were suspended in ethanol (30 mL), and the obtained suspension was refluxed at 70°C for 36 hours. After the resultant mixture was concentrated, cold acetone was added to the concentrated mixture, and the suction filtration was performed. The residue was washed with cold acetone, and the residue was dried under vacuum to obtain the target product 2 as yellow powder (yield: 1,734 mg, 61%).
¹H-NMR (400 MHz, DMSO-d₆) δ 9.23 (s, 1H), 8.89 (s, 2H), 8.33 (d, 2H, J = 29.3 Hz), 8.19 (d, 2H, J = 29.3 Hz).
¹³C-NMR (100 MHz, DMSO-d₆) δ 165.2, 146.5, 146.2, 144.3, 139.8, 125.7, 124.3, 113.8.
MS(ESI): m/z, C₁₀H₆N₅O₄S₂⁻, calculation value: 324.0, and measurement value: 324.0 ([M-H]⁻).

### (3) Synthesis of 6-(4-aminophenyl)imidazo[2,1-b][1,3,4]thiadiazole-2-sulfonamide (compound 3)

A compound 3 (325 mg, 1 mmol) was suspended in ethanol (30 mL), and tin(II) chloride (anhydrous) (1,896 mg, 10 mmol) was added to the obtained suspension. After the obtained mixture was refluxed at 70°C overnight, the resultant mixture was concentrated, the concentrated mixture was neutralized with a saturated aqueous solution of NaHCO₃, and then the neutralized mixture was extracted with ethyl acetate (50 mL × 3). The organic layer was washed with saturated saline and dried over sodium sulfate, and then the solvent was distilled off, and the residue was dried under vacuum to obtain the target product 3 as yellow powder (yield: 125 mg, 42%).
¹H-NMR (400 MHz, DMSO-d₆) δ 8.69 (s, 2H), 8.56 (s, 1H), 7.57 (d, 2H, J = 19.7 Hz), 6.60 (d, 2H, J = 19.7 Hz), 5.29 (s, 2H).
¹³C-NMR (100 MHz, DMSO-d₆) δ 162.7, 148.9, 148.2, 144.6, 126.2, 121.0, 114.0, 108.2.
MS (APCI) : m/z, C₁₀H₁₀N₅O₂S₂⁺, calculation value: 296.0, and measurement value: 296.0 ([M+H]⁺).

### Example 7: Synthesis of ITDA1 and [^{113/115}In] ITDA1

The labeling precursor compound represented by the above formula (2-1), that is, 2,2',2''-(10-(2-oxo-2-((4-(2-sulfamoylimidazo[2,1-b] [1,3,4]thiadiazol-6-yl)phenyl)amino)ethyl)-1,4,7,10-tetraazacyclododecan-1,4,7-triyl) triacetic acid (referred to as "ITDA1" in the present specification), and [^{113/115}In] ITDA1 that is a complex of the ITDA1 with indium-113/115 were synthesized in accordance with the following scheme.

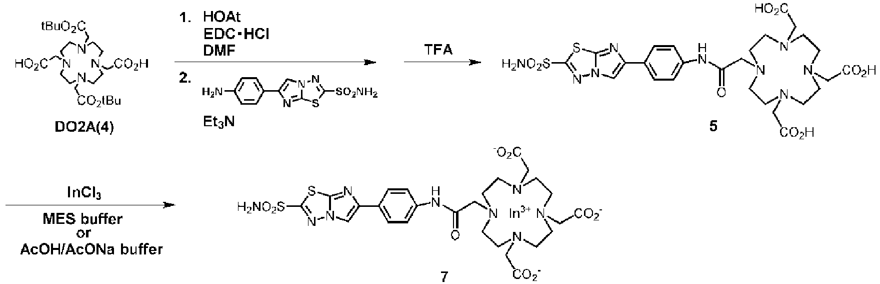

### (1) Synthesis of 2,2'-(4,10-bis(2-(tert-butoxy)-2-oxoethyl)-1,4,7,10-tetraazacyclododecane-1,7-diyl) diacetic acid (DO2A) (compound 4)

The compound 4 was synthesized in 5 steps in accordance with a previously published paper (Mukai T et al. Bioorg Med Chem. 2009; 17 (13): 4285-9. Design of Ga-DOTA-based bifunctional radiopharmaceuticals: Two functional moieties can be conjugated to radiogallium DOTA without reducing the complex stability).

### (2) Synthesis of 2,2',2''-(10-(2-oxo-2-((4-(2-sulfamoylimidazo[2,1-b][1,3,4]thiadiazol-6-yl)phenyl)amino)ethyl)-1,4,7,10-tetraazacyclododecan-1,4,7-triyl) triacetic acid (ITDA1) (compound 5)

A compound 4 (105 mg, 0.20 mmol), 1-ethyl-3-(3-dimethylaminopropyl)carbodiimide hydrochloride (EDC·HCl) (38 mg, 0.20 mmol), and 1-hydroxybenzotriazole (HOAt) (27 mg, 0.20 mmol) were dissolved in anhydrous N,N-dimethylformamide (DMF, 4 mL) in an ice bath. The obtained solution was stirred for 30 minutes while cooling in ice under an argon atmosphere. Into the resultant solution, a compound 3 (60 mg, 0.20 mmol), anhydrous DMF (4 mL), and triethylamine (28 µL, 0.20 mmol) were added, and then the obtained mixture was stirred at room temperature for 48 hours under an argon atmosphere. The resultant mixture was freeze-dried, trifluoroacetic acid (4 mL) was slowly added to the residue in an ice bath, and the obtained mixture was stirred for 7 hours at room temperature. After the concentration, the concentrated mixture was dissolved in dimethyl sulfoxide (DMSO), and then the obtained solution was purified by reversed-phase HPLC to obtain the target product 5.

Purification conditions (1st): Cosmosil AR-II column (20 × 250 mm), mobile phase: acetonitrile/water/trifluoroacetic acid [10/90/0.1 (5 min) → 25/75/0.1 (75 min)], and flow rate: 5 mL/min.

Purification conditions (2nd): Cosmosil AR-II column (10 × 250 mm), mobile phase: acetonitrile/water/trifluoroacetic acid [15/85/0.1], and flow rate: 2.8 mL/min.
¹H-NMR (400 MHz, DMSO-d₆) δ 10.59 (s, 1H), 8.81 (s, 1H), 8.75 (s, 2H), 7.86 (d, 2H, J = 21.5 Hz), 7.65 (d, 2H, J = 21.5 Hz), 3.9-4.1 (br, 16H), 3.3-3.1 (br, 8H).
MS(ESI) : m/z, C₂₆H₃₆N₉O₉S₂⁺, calculation value: 682.2, and measurement value: 682.2 ([M+H]⁺).

### (3) Synthesis of [^{113/115}In] indium(III) 2, 2', 2''-(10-(2-oxo-2-((4-(2-sulfamoylimidazo[2,1-b] [1,3,4]thiadiazol-6-yl)phenyl)amino)ethyl)-1,4,7,10-tetraazacyclododecan-1,4,7-triyl) triacetic acid ([^{113/115}In]ITDA1) (compound 7)

Into the compound 5 (4.8 mg, 0.007 mmol) being dissolved in dimethyl sulfoxide, anhydrous [^{113/115}In]indium(III) chloride (15.5 mg, 0.07 mmol), and a 0.1M MES buffer solution (pH 5.5, 7 mL) were added, and the obtained mixture was stirred at 60°C for 24 hours. After the concentration, the residue was dissolved in dimethyl sulfoxide, and the obtained solution was purified by reversed-phase HPLC to obtain the target product 7 (yield: 4.2 mg, 76%).

Purification conditions: Cosmosil AR-II column (10 × 250 mm), mobile phase: acetonitrile/water/trifluoroacetic acid [5/95/0.1 (5 min) → 35/65/0.1 (95 min)], flow rate: 4 mL/min, and retention time: 34 min.
¹H-NMR (400 MHz, DMSO-d₆) δ 10.91 (s, 1H), 8.84 (s, 1H), 8.74 (s, 2H), 7.87 (d, 2H, J = 14.8 Hz), 7.64 (d, 2H, J = 14.8 Hz), 3.4-3.3 (br, 4H), 3.2-3.2 (br, 12H), 3.0-2.8 (br, 8H).
MS (ESI) : m/z, C₂₆H₃₃InN₉O₉S₂⁺, calculation value: 794.1, and measurement value: 794.1 ([M+H]⁺).

### Example 8: Synthesis of ITDA2 and [^{113/115}In] ITDA2

The labeling precursor compound represented by the above formula (2-2), that is, 2,2'-(4,10-bis(2-oxo-2-((4-(2-sulfamoylimidazo[2,1-b] [1,3,4]thiadiazol-6-yl)phenyl)amino)ethyl)-1,4,7,10-tetraazacyclododecan-1,7-diyl) diacetic acid (referred to as "ITDA2" in the present specification) was synthesized in accordance with the following scheme.

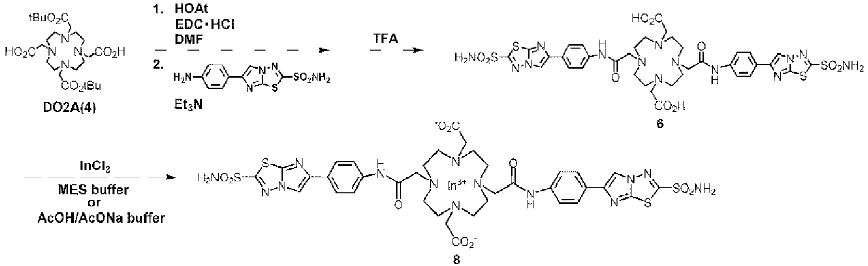

### (1) Synthesis of 2,2'-(4,10-bis(2-oxo-2-((4-(2-sulfamoylimidazo[2,1-b][1,3,4]thiadiazol-6-yl)phenyl)amino)ethyl)-1,4,7,10-tetraazacyclododecan-1,7-diyl) diacetic acid (ITDA2) (compound 6)

The compound 4 (108 mg, 0.21 mmol), 1-ethyl-3-(3-dimethylaminopropyl)carbodiimide hydrochloride (EDC·HCl) (80 mg, 0.42 mmol), 1-hydroxybenzotriazole (HOAt) (57 mg, 0.42 mmol) were dissolved in anhydrous DMF (5 mL) in an ice bath. The obtained solution was stirred for 30 minutes while cooling in ice under an argon atmosphere. Into the resultant mixture, a compound 3 (126 mg, 0.42 mmol), anhydrous DMF (5 mL), and triethylamine (58 µL, 0.42 mmol) were added, and then the obtained mixture was stirred at room temperature for 26 hours under an argon atmosphere. The resultant mixture was freeze-dried, trifluoroacetic acid (8 mL) was slowly added to the residue in an ice bath, and the obtained mixture was stirred at room temperature for 6 hours. After the concentration, the concentrated mixture was dissolved in dimethyl sulfoxide, and the obtained solution was purified by reversed-phase HPLC to obtain the target product 6.

Purification conditions (1st): Cosmosil AR-II column (10 × 250 mm), mobile phase: acetonitrile/water/trifluoroacetic acid [5/95/0.1 (5 min) → 35/65/0.1 (35 min)], and flow rate: 4 mL/min.

Purification conditions (2nd): Cosmosil AR-II column (10 × 250 mm), mobile phase: acetonitrile/water/trifluoroacetic acid [5/95/0.1 (5 min) → 35/65/0.1 (95 min)], and flow rate: 4 mL/min.
¹H-NMR (400 MHz, DMSO-d₆) δ 10.65 (s, 2H), 8.78 (s, 1H), 8.74 (s, 2H), 7.84 (d, 2H, J = 22.6 Hz), 7.67 (d, 2H, J = 22.6 Hz), 4.16 (br, 4H), 3.70 (br, 4H), 3.46 (br, 8H), 3.20 (br, 8H).
MS (ESI) : m/z, C₃₆H₄₃N₁₄O₁₀S₄⁺, calculation value: 959.2, and measurement value: 959.2 ([M+H]⁺).

### (2) Synthesis of [^{113/115}In]indium (III)2, 2'-(4, 10-bis(2-oxo-2-((4-(2-sulfamoylimidazo[2,1-b] [1,3,4]thiadiazol-6-yl)phenyl)amino)ethyl)-1,4,7,10-tetraazacyclododecan-1,7-diyl) diacetic acid ([^{113/115}In]ITDA2) (compound 8)

Into the compound 6 (4.5 mg, 0.004 mmol) being dissolved in dimethyl sulfoxide, anhydrous [^{113/115}In] indium (III) chloride (10 mg, 0.04 mmol), and a 0.1M MES buffer solution (pH 5.5, 4 mL) were added, and the obtained mixture was stirred at 60°C for 16 hours. After the concentration, the residue was dissolved in dimethyl sulfoxide, and the obtained solution was purified by reversed-phase HPLC to obtain the target product 8.

Purification conditions: Cosmosil AR-II column (10 × 250 mm), mobile phase: acetonitrile/water/trifluoroacetic acid [5/95/0.1 (5 min) → 35/65/0.1 (35 min)], flow rate: 4 mL/min, and retention time: 30.5 min.
¹H-NMR (400 MHz, DMSO-d₆) δ 11.04 (s, 2H), 8.85 (s, 2H), 8.72 (s, 4H), 7.87 (d, 4H, J = 19.1 Hz), 7.63 (d, 4H, J = 19.1 Hz), 2.6-3.0 (br, 24H).
MS (ESI) : m/z, C₃₆H₄₀InN₁₄O₁₀S₄⁺, calculation value: 1071.1, and measurement value: 1071.2 ([M+H]⁺).

### Example 9: Synthesis of [¹¹¹In] ITDA1 and [¹¹¹In] ITDA2

### (1) Synthesis of [¹¹¹In] ITDA1

A complex of the compound represented by the above formula (2-1) with ¹¹¹In, that is, [¹¹¹In]indium(III)2,2',2''-(10-(2-oxo-2-((4-(2-sulfamoylimidazo[2,1-b] [1,3,4]thiadiazol-6-yl)phenyl)amino)ethyl)-1,4,7,10-tetraazacyclododecan-1,4,7-triyl) triacetic acid (referred to as "[¹¹¹In]ITDA1" in the present specification) was synthesized in accordance with the following scheme.

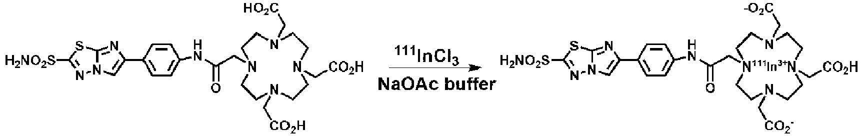

A sodium acetate buffer solution (0.1 M, pH 4.6, 200 µL) and [¹¹¹In]indium chloride (6.9 MBq in 100 µL saline solution) were incubated under room temperature for 10 minutes in a low protein binding tube (1.5 mL), and then into the incubated mixture, the ITDA1 (0.13 mg, 200 nmol in 10 µL dimethyl sulfoxide) obtained in Example 7 was added, and the obtained mixture was incubated at 90°C for 30 minutes. After that, the resultant mixture was purified by reversed-phase high performance liquid chromatography (RP-HPLC) to obtain [¹¹¹In]ITDA1 of 5.3 MBq.

The obtained [¹¹¹In]ITDA1 was analyzed by RP-HPLC under the following conditions. As a result, the radiochemical yield of the obtained [¹¹¹In]ITDA1 was 76.4%, and the radiochemical purity of the [¹¹¹In]ITDA1 was > 99%.

### <RP-HPLC conditions>

Column: Cosmosil 5C₁₈-AR-II 4.6 ID × 150 mm;
Flow rate: 0.6 mL/min;
Mobile phase: Acetonitrile/water/trifluoroacetic acid = 10/90/0.1 (0 min) → 40/60/0.1 (30 min)

### (2) Examination of synthesis conditions of [¹¹¹In]ITDA2

The synthesis conditions of a complex of the compound represented by the above formula (2-2) with ¹¹¹In, that is, [¹¹¹In] indium(III)2,2'-(4,10-bis(2-oxo-2-((4-(2-sulfamoylimidazo[2,1-b] [1,3,4]thiadiazol-6-yl)phenyl)amino)ethyl)-1,4,7,10-tetraazacyclododecan-1,7-diyl) diacetic acid (referred to as "[¹¹¹In]ITDA2" in the present specification) were examined as follows.

Into a MES buffer solution (0.1 M, pH 5.6, 200 µL) or a sodium acetate buffer solution (0.1 M, pH 4.6, 200 µL), [¹¹¹In] indium chloride (2.6 to 5.9 MBq in 100 µL of saline solution) was added, and the obtained mixture was incubated under room temperature for 10 minutes, and then into the incubated mixture, the ITDA2 (0.05 mg, 50 nmol in 10 µL dimethyl sulfoxide) obtained in Example 8 was added, and the obtained mixture was incubated at the temperatures and times shown in Table 2 by using a heat block or microwave. After that, the mixture was purified by reversed-phase high performance liquid chromatography (RP-HPLC) to obtain 0.6 to 1.0 MBq of [¹¹¹In]ITDA2.

The obtained [¹¹¹In]ITDA2 was analyzed by RP-HPLC under the following conditions. As a result, the radiochemical yields of the obtained [¹¹¹In]ITDA2 were as shown in Table 2.

### <RP-HPLC conditions>

Column: Cosmosil 5C₁₈-AR-II 4.6 ID × 150 mm;
Flow rate: 1.0 mL/min;
Mobile phase: Acetonitrile/water/trifluoroacetic acid = 10/90/0.1 (0 mmin) → 40/60/0.1 (60 min)

**Table 2**

| | Heat block heating | Microwave heating | | |
|---|---|---|---|---|
| Incubation conditions | 90°C 30 min | 90°C 1 min | 90°C 2 min | 90°C 10 min |
| Sodium acetate buffer solution | 17.4% | 22.0% | 27.2% | 24.0% |
| MES buffer solution | 15.8 - 18.8% | | | |

### (3) Synthesis of [¹¹¹In]ITDA2

[¹¹¹In]ITDA2 was synthesized in accordance with the following scheme.

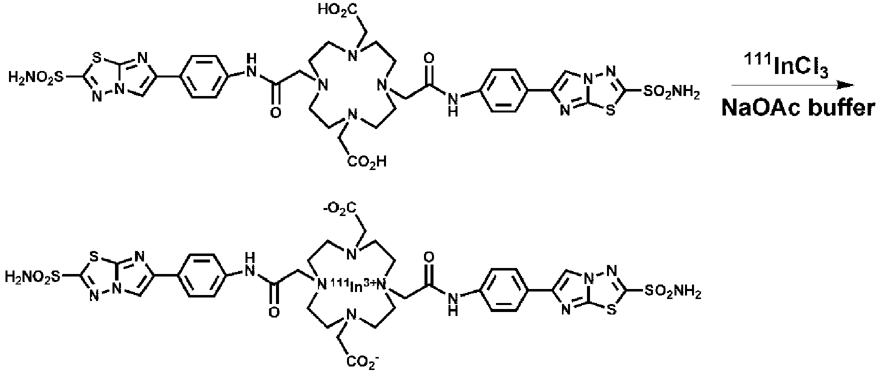

A sodium acetate buffer solution (0.1 M, pH 4.6, 200 µL) and [¹¹¹In]indium chloride (2.4 MBq in 100 µL saline solution) were incubated for 10 minutes under room temperature in a low protein binding tube (1.5 mL), and then into the incubated mixture, the ITDA2 (0.05 mg, 50 nmol in 10 µL dimethyl sulfoxide) obtained in Example 8 and dimethyl sulfoxide (190 µL) were added, and the obtained mixture was incubated at 90°C for 30 minutes by a heat block. After that, the resultant mixture was purified by reversed-phase high performance liquid chromatography (RP-HPLC) under the following conditions to obtain 1.5 MBq of [¹¹¹In]ITDA2.

The obtained [¹¹¹In]ITDA2 was analyzed by RP-HPLC under the following conditions. As a result, the radiochemical yield of the obtained [¹¹¹In]ITDA2 was 64.5%, and the radiochemical purity of the [¹¹¹In]ITDA2 was >99%.

### <RP-HPLC conditions>

Column: Cosmosil 5C₁₈-AR-II 4.6 ID × 150 mm;
Flow rate: 1.0 mL/min;
Mobile phase: Acetonitrile/water/trifluoroacetic acid =
   10/90/0.1 (0 min) → 40/60/0.1 (60 min) (in a case of purification)
   10/90/0.1 (0 min) → 40/60/0.1 (30 min) (in a case of analysis)

### Example 10: Synthesis of ITDA3 and [^{113/115}In] ITDA3

The labeling precursor compound represented by the above formula (3-1) (wherein R₄ represents CO₂H), that is, 2,2',2"-(10-(1-carboxy-4-oxo-4-((4-(2-sulfamoylimidazo[2,1-b] [1,3,4]thiadiazol-6-yl)phenyl)amino)butyl)-1,4,7,10-tetraazacyclododecan-1,4,7-triyl) triacetic acid (referred to as "ITDA3" in the present specification), and [^{113/115}In] ITDA3 that is a complex of the ITDA3 with indium-113/115 were synthesized in accordance with the following scheme.

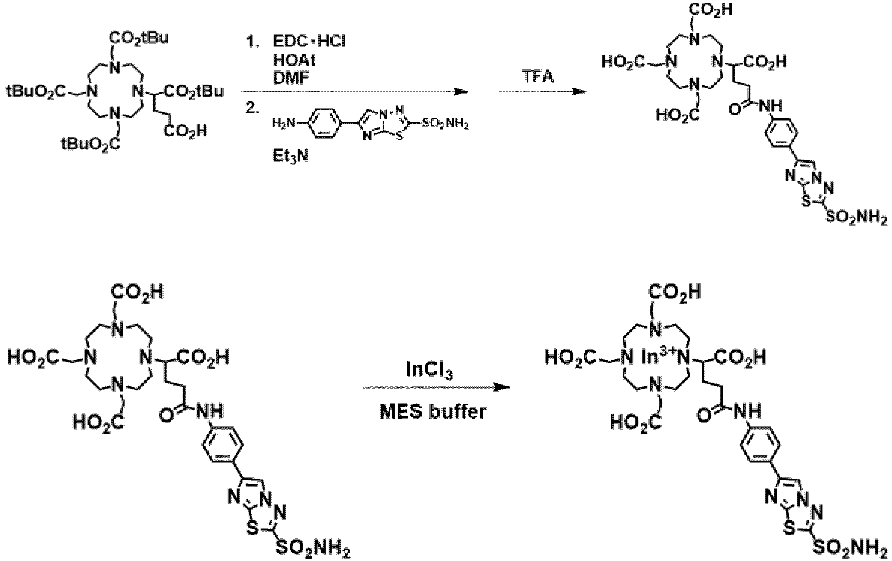

### (1) Synthesis of 5-(tert-butoxy)-5-oxo-4-(4,7,10-tris(2-(tert-butoxy)-2-oxoethyl)-1,4,7,10-tetraazacyclododecan-1-yl) pentanoic acid (DOTAGA)

DOTAGA was synthesized in accordance with a previously published paper (Eisenwiener KP et al. Bioorg Med Chem Lett. 2000; 10 (18): 2133-5. A convenient synthesis of novel bifunctional prochelators for coupling to bioactive peptides for radiometal labelling).

### (2) Synthesis of ITDA3

DOTAGA (65 mg, 0.093 mmol), 1-ethyl-3-(3-dimethylaminopropyl)carbodiimide hydrochloride (EDC·HCl) (18 mg, 0.093 mmol), and 1-hydroxybenzotriazole (HOAt) (13 mg, 0.093 mmol) were dissolved in anhydrous N,N-dimethylformamide (4 mL) in an ice bath. The obtained solution was stirred for 30 minutes in an ice bath under an argon atmosphere. Into the resultant mixture, 6-(4-aminophenyl)imidazo[2,1-b][1,3,4]thiadiazole-2-sulfonamide (compound 3) (27 mg, 0.093 mmol), and triethylamine (13µL, 0.093 mmol) were added, and then the obtained mixture was stirred at 50°C for 6.5 days under an argon atmosphere. The resultant mixture was freeze-dried, trifluoroacetic acid (4 mL) was slowly added to the residue in an ice bath, and the obtained mixture was stirred at room temperature for 6 hours. After the concentration, the concentrated mixture was dissolved in N,N-dimethyl sulfoxide, and the obtained solution was purified by reversed-phase high performance liquid chromatography.

Purification conditions (1st): Cosmosil AR-II column (20 × 250 mm), mobile phase: MeCN/H₂O/TFA [10/90/0.1 (5 min) → 40/60/0.1 (65 min)], and flow rate: 5 mL/min.

Purification conditions (2nd): Cosmosil AR-II column (10 × 250 mm), mobile phase: MeCN/H₂O/TFA [14/86/0.1 (isocratic)], and flow rate: 4 mL/min.

Purification conditions (3rd): Cosmosil AR-II column (4.6 ID × 150 mm), mobile phase: MeCN/H₂O/TFA [18/82/0.1 (isocratic)], and flow rate: 0.6 mL/min.
Yield: 1.5 mg (2%)
¹H-NMR (400 MHz, DMSO-d₆) δ 10.11 (s, 1H), 8.79 (s, 1H), 8.75 (s, 2H), 7.84 (d, 2H, J = 25.2 Hz), 7.66 (d, 2H, J = 25.2 Hz), 3.6-2.3 (br, 27H).
MS(ESI): m/z, C₂₉H₃₉N₉O₁₁S₂, calculation value: 753.2, and measurement value: 754.2 ([M+H]⁺).

### (3) Synthesis of [^{113/115}In] ITDA3

Into ITDA3 (4 mg, 0.005 mmol) being dissolved in N,N-dimethyl sulfoxide, anhydrous indium chloride (III) (11.7 mg, 0.05 mmol), and a 0.1M MES buffer (pH 5.5, 5 mL) were added, and the obtained mixture was stirred at 60°C for 24 hours. After the concentration, the residue was dissolved in N,N-dimethyl sulfoxide, and the obtained solution was purified by reversed-phase high performance liquid chromatography.
MS (ESI) : m/z, C₂₉H₃₆InN₉O₁₁S₂, calculation value: 865.1, and measurement value: 866.1 ([M+H]⁺).

### (4) Synthesis of [¹¹¹In] ITDA3

An acetic acid/sodium acetate buffer (0.1 M, pH 4.6, 200 µL) and ¹¹¹InCl₃ (100 µL) were put into a low protein binding Eppendorf tube (1.5 mL), and the incubation was performed at room temperature for 10 minutes. After that, into the resultant mixture, ITDA3 (10 µL DMSO solution) was added. The obtained mixture was incubated at 90°C for 30 minutes by using a block heater, and the resultant mixture was purified by reversed-phase high performance liquid chromatography.

Purification conditions: Cosmosil AR-II column (4.6 ID × 150 mm), mobile phase: MeCN/H₂O/TFA [10/90/0.1 (0 min) → 40/60/0.1 (30 min)], and flow rate: 1.0 mL/MIN.

The radiochemical purity was confirmed by reversed-phase high performance liquid chromatography in combination with the unlabeled compound. [¹¹¹In]ITDA3 was obtained with a radiochemical yield of 57.3% and a radiochemical purity of 95% or more.

### Example 11: In vitro CA-IX affinity and specificity of [ ¹¹¹In] ITDA1, [¹¹¹In] ITDA2, and [¹¹¹In] ITDA3 under roomtemperature condition

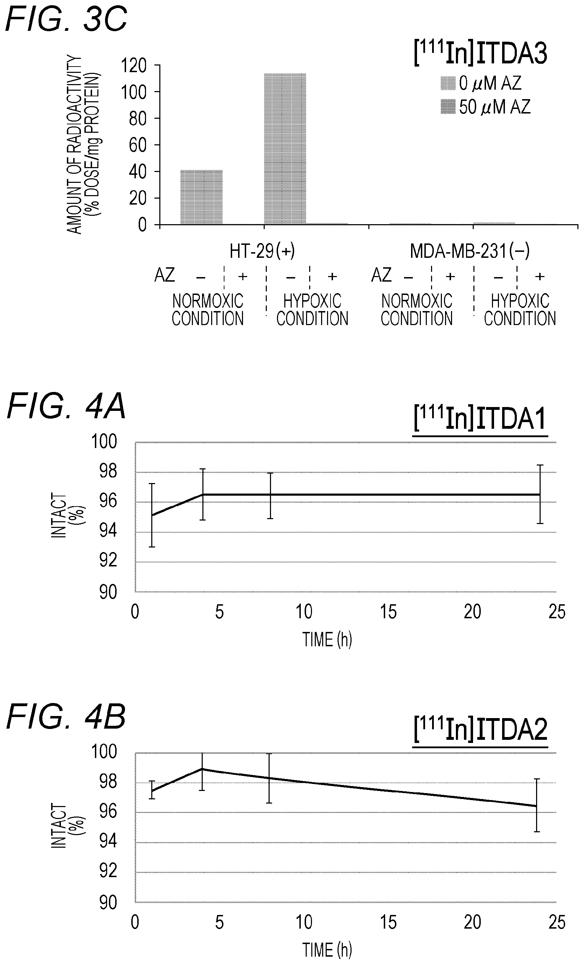

HT-29 cells (purchased from Dainippon Sumitomo Pharma Co., Ltd.) and MDA-MB-231 cells (purchased from Dainippon Sumitomo Pharma Co., Ltd.) were incubated in a 12-well plate (2.0×10⁵ cells/well) at 37°C for 24 hours under an atmosphere of 5% carbon dioxide and 21% oxygen, and then further incubated at 37°C for 24 hours under an atmosphere of 5% carbon dioxide and 21% or 1% oxygen. After the culture medium was removed, Dulbecco's modified Eagle's medium (DMEM, 1 mL) containing [¹¹¹In]ITDA1 (14 kBq), [¹¹¹In] ITDA2 (26 kBq), or [¹¹¹In]ITDA3 (37 kBq) was added to each well, and then the incubation was performed at 37°C for 2 hours under an atmosphere of 5% carbon dioxide and 21% or 1% oxygen. In addition, a separate plate was incubated in a similar manner as in the above except that acetazolamide (AZ, 50 µM) was added to [¹¹¹In]ITDA1 (14 kBq), [¹¹¹In]ITDA2 (26 kBq), or [¹¹¹In] ITDA3 (37 kBq) as a competitive compound for CA-IX. After that, each well was washed with 1 mL of phosphate-buffered saline (PBS, pH 7.4) (manufactured by NACALAI TESQUE, INC.), and the cells were lysed at room temperature in an aqueous solution of 1 N sodium hydroxide ([¹¹¹In]ITDA1: 0.25 mL × 2, [¹¹¹In]ITDA2: 0.5 mL × 2, and [¹¹¹In] ITDA3: 0.2 mL × 2). The amount of radioactivity bound to the cells was measured by a gamma counter (model name: Wallac 1470 Wizard, PerkinElmer, Massachusetts, made in U.S.A., the same applies to the following examples). The results are shown in Figs. 3A, 3B, and 3C.

Fig. 3A is a graph showing whether or not the affinity of compound [¹¹¹In]ITDA1 for CA-IX in each of the HT-29 cells and the MDA-MB-231 cells is inhibited by the coexistence of acetazolamide (AZ). Further, Fig. 3B is a graph showing whether or not the affinity of compound [¹¹¹In]ITDA2 for CA-IX in each of the HT-29 cells and the MDA-MB-231 cells is inhibited by the coexistence of acetazolamide (AZ). Moreover, Fig. 3C is a graph showing whether or not the affinity of compound [¹¹¹In]ITDA3 for CA-IX in each of the HT-29 cells and the MDA-MB-231 cells is inhibited by the coexistence of acetazolamide (AZ).

From Figs. 3A, 3B, and 3C, the inhibition was observed in the presence of acetazolamide under both normoxic and hypoxic conditions in the CA-IX-positive HT-29 cells, but the inhibition was not observed in the absence of the competitive compound. On the other hand, the competitive inhibition was not observed regardless of the presence or absence of acetazolamide under both normoxic and hypoxic conditions in the CA-IX-negative MDA-MB-231 cells. From the above results, it can be understood that [¹¹¹In]ITDA1, [¹¹¹In]ITDA2, and [¹¹¹In]ITDA3 selectively bind to the CA-IX.

### Example 12: Measurement of partition coefficient of [¹¹¹In]ITDA1, [¹¹¹In]ITDA2, and [¹¹¹In]ITDA3

The 1-octanol/PBS (pH 7.4) partition coefficients of [¹¹¹In]ITDA1 and [¹¹¹In]ITDA2, which were obtained in Example 6, and [¹¹¹In]ITDA3 obtained in Example 10 were measured.

The above two phases were saturated with each other in advance, and 1-octanol (3 mL) and PBS (3 mL) were pipetted into a 15-mL test tube containing [¹¹¹In]ITDA1 (37 kBq), [¹¹¹In]ITDA2 (37 kBq), or [¹¹¹In]ITDA3 (370 kBq). The test tube was vortexed for 2 minutes and centrifuged (4,000 g, 5 minutes). 0.5 mL was taken from each of the 1-octanol phase and the PBS phase, and transferred to two test tubes, respectively for measurement. The residual PBS phase (1 mL), and newly prepared 1-octanol (3 mL) and PBS (2 mL) were pipetted into a new test tube. The amount of radioactivity in each test tube was measured by using a gamma counter. The partition coefficient was calculated by using the equation logP_{ow} = log₁₀[count_{1-octanol}/count_{PBS}]. As a result, the logP_{ow} of [¹¹¹In]ITDA1 was -3.72 ± 0.05, the logP_{ow} of [¹¹¹In]ITDA2 was -2.32 ± 0.01, and the logP_{ow} of [¹¹¹_{In}]ITDA3 was -3.47 ± 0.09.

### Example 13: In vitro stability of [¹¹¹In] ITDA1, [¹¹¹In] ITDA2, and [¹¹¹In] ITDA3 in mouse plasma

Blood was collected from a ddy mouse (purchased from Shimizu Laboratory Supplies Co., Ltd.), and centrifuged (1,200 g, 10 minutes) in a venous blood collection tube (manufactured by Becton, Dickinson and Company). The blood plasma (200 µL) was separated, a saline solution (185 kBq) of [¹¹¹In] ITDA1, [¹¹¹In] ITDA2, or [¹¹¹In]ITDA3 was added into the blood plasma, and the obtained mixture was incubated at 37°C for 1, 4, 8, and for 24 hours (n = 3). Further, into the incubated mixture, acetonitrile (200 µL) was added, and then the mixture was centrifuged (10,000 g, 5 minutes), the supernatant was filtered with COSMONICE Filter (S) (0.45 µm, 4mm) (manufactured by NACALAI TESQUE, INC.), and the filtrate was analyzed by RP-HPLC under the following conditions. The results are shown in Figs. 4A and 4B. In this regard, the intact % in Figs. 4A and 4B was determined from the area percentage of the RP-HPLC elution pattern.

### <RP-HPLC conditions>

Column: Cosmosil C₁₈ column (5C₁₈-PAQ, 4.6 × 250 mm)
Mobile phase: water/acetonitrile/trifluoroacetic acid (90 : 10 : 0.1 (0 min) → 60 : 40 : 0.1 (30 min)), and flow rate for analysis: 1.0 mL/min

From Figs. 4A and 4B, it can be understood that [¹¹¹In] ITDA1 and [¹¹¹In] ITDA2 have sufficient stability during 24-hour incubation. The results of [¹¹¹In]ITDA3 are shown in Table 3. From Table 3, [¹¹¹In] ITDA3 showed intact 95% or more stability during the incubation for 1 to 24 hours, and showed almost the same results as those of [¹¹¹In]ITDA1.

**Table 3**

| Time (h) | 1 | 4 | 8 | 24 |
|---|---|---|---|---|
| Stability (intact (%)) | 97.4 ± 0.8 | 99.0 ± 0.6 | 98.0 ± 0.1 | 98.8 ± 0.4 |

### Example 14: Biodistribution of [¹¹¹In]ITDA1 in cancer-bearing mouse

HT-29 cells (5×10⁶ cells/mouse) were subcutaneously inoculated into the left shoulder of each of 5-week old male BALB/c nude mice (purchased from Shimizu Laboratory Supplies Co., Ltd., the same applies to the following Examples). A saline solution (100 µL) in which [¹¹¹In]ITDA1 (37 kBq) had been dissolved was administered to each of the HT-29 cancer-bearing mice (n = 5) via the tail vein. The mice were slaughtered after the lapse of 1, 4, 8, and 24 hours from the administration, and the blood, spleen, pancreas, stomach, intestines, kidney, liver, heart, lungs, brain, HT-29-transplanted tumor, and muscle were recovered. The weight of each organ was measured, and the amount of radioactivity in each organ was measured by using a gamma counter. The accumulation of radioactivity in each organ was expressed by % ID (injected dose)/g obtained by dividing the ratio of the amount of radioactivity of each organ to the administered amount of radioactivity by the organ weight. The results are shown in Table 4.

**Table 4**

| Organ | Time (h) after administration | | | |
|---|---|---|---|---|
| | 1 | 4 | 8 | 24 |
| Blood | 0.27 ± 0.03 | 0.25 ± 0.18 | 0.18 ± 0.02 | 0.16 ± 0.02 |
| Spleen | 0.33 ± 0.10 | 0.31 ± 0.12 | 0.21 ± 0.05 | 0.42 ± 0.18 |
| Pancreas | 31.32 ± 5.29 | 18.81 ± 2.04 | 9.80 ± 2.34 | 8.13 ± 0.91 |
| Stomach | 2.59 ± 0.69 | 5.22 ± 0.79 | 6.09 ± 0.82 | 8.74 ± 0.46 |
| Intestines | 10.86 ± 1.27 | 14.20 ± 1.28 | 14.99 ± 1.27 | 17.99 ± 1.59 |
| Kidney | 111.20 ± 12.39 | 115.12 ± 8.70 | 83.81 ± 7.88 | 65.13 ± 7.13 |
| Liver | 3.59 ± 0.98 | 3.86 ± 0.92 | 3.15 ± 0.78 | 2.21 ± 0.34 |
| Heart | 7.58 ± 1.20 | 3.49 ± 0.74 | 2.16 ± 0.31 | 1.56 ± 0.30 |
| Lungs | 13.49 ± 5.52 | 5.09 ± 1.69 | 3.54 ± 1.19 | 2.06 ± 0.66 |
| Brain n | 0.30 ± 0.02 | 0.35 ± 0.05 | 0.41 ± 0.05 | 0.48 ± 0.03 |
| Tumor (HT-29) | 3.81 ± 0.79 | 4.20 ± 0.68 | 5.16 ± 0.62 | 8.71 ± 1.41 |
| Muscle | 5.44 ± 1.59 | 2.69 ± 0.76 | 1.75 ± 0.37 | 1.43 ± 0.53 |
| Tumor/blood | 14.41 ± 3.35 | 21.70 ± 8.65 | 29.14 ± 5.15 | 53.56 ± 5.53 |
| Tumor/muscle | 0.74 ± 0.25 | 1.62 ± 0.33 | 3.03 ± 0.66 | 6.59 ± 2.18 |

| | | | | |
|---|---|---|---|---|
| Note: In Table 4, the unit of the numerical value is % ID/tissue weight (g), and is shown by the mean ± standard deviation of the samples. The unit of the numerical value of the stomach is % ID. ^{†}p < 0.05 vs. 24 h. | | | | |

As shown in Table 4, [¹¹¹In]ITDA1 accumulates in a large amount in the HT-29-transplanted tumor (3.81% ID/g one hour after administration), and therefore, it can be understood that [¹¹¹In]ITDA1 has high specificity for a tumor expressing the CA-IX. Further, it is considered that the amount of [¹¹¹In]ITDA1 to be incorporated into the HT-29-transplanted tumor is larger than that of the conventional probe for CA-IX visualization. In addition, in Table 4, both of the HT-29-transplanted tumor/blood ratio and the HT-29-transplanted tumor/muscle ratio increased with time, and reached 53.56 and 6.59, respectively, after the lapse of 24 hours from the administration, and therefore, it can be understood that [¹¹¹In]ITDA1 has favorable pharmacokinetics for in vivo imaging of solid tumors.

### Example 15: CA-IX selectivity of [¹¹¹In]ITDA1 in cancer-bearing mouse

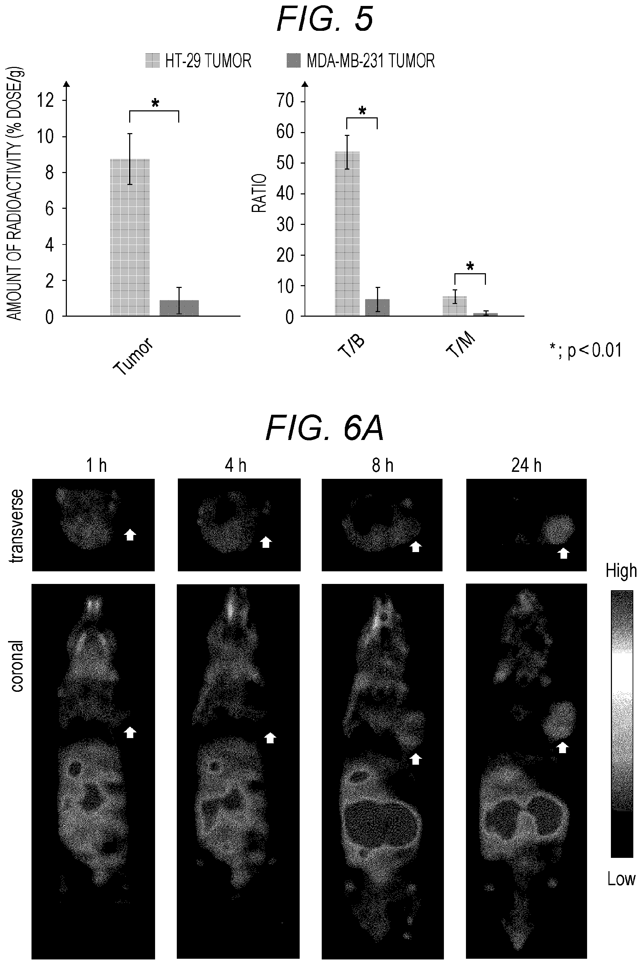

HT-29 cells (5×10⁶ cells/mouse) and MDA-MB-231 cells (5×10⁶ cells/mouse) were subcutaneously inoculated into the left shoulders of 5-week old male BALB/c nude mice, respectively. A saline solution (100 µL) in which [¹¹¹In]ITDA1 (37 kBq) had been dissolved was administered to each of the cancer-bearing mice (n = 5) via the tail vein. The mice were slaughtered after the lapse of 24 hours from the administration, and the blood, transplanted tumor, and muscle were recovered from each of the mice. The weight of each organ was measured, and the amount of radioactivity in each organ was measured by using a gamma counter. The accumulation of radioactivity in each organ was expressed by % ID (injected dose)/g obtained by dividing the ratio of the amount of radioactivity of each organ to the administered amount of radioactivity by the organ weight. The results are shown in Fig. 5. In the drawing, the symbol "*" indicates the data which were obtained by performing the testing using Welch's t-test as the test method at the 1% significance level and showed a significant difference.

As shown in Table 5, a significant difference was observed between the amount of [¹¹¹In]ITDA1 accumulated in the HT-29 tumor and the amount of [¹¹¹In]ITDA1 accumulated in the MDA-MB-231 tumor. A significant difference was similarly observed also in the transplanted tumor/blood ratio and the transplanted tumor/muscle ratio. From these results, CA-IX selective tumor accumulation of [¹¹¹In]ITDA1 was confirmed.

### Example 16: SPECT/CT imaging using [¹¹¹In]ITDA1

HT-29 cells (5×10⁶ cells/mouse) and MDA-MB-231 cells (5×10⁶ cells/mouse) were subcutaneously inoculated into the left shoulders of 5-week old male BALB/c nude mice, respectively. A saline solution (150 µL) in which [¹¹¹In]ITDA1 (23.8 to 26.2MBq) had been dissolved was administered to each of the cancer-bearing mice via the tail vein. Each of the mice was subjected to SPECT/CT imaging after the lapse of 1, 4, 8, and 24 hours from the administration. U-SPECT-II/CT (single-pinhole collimator 0.6 or 1.0 mm, resolution: 0.45 mm) manufactured by Milab was used as the SPECT/CT device, and data were collected for 60 minutes in 1, 4, 8, and 24 hours from the administration.

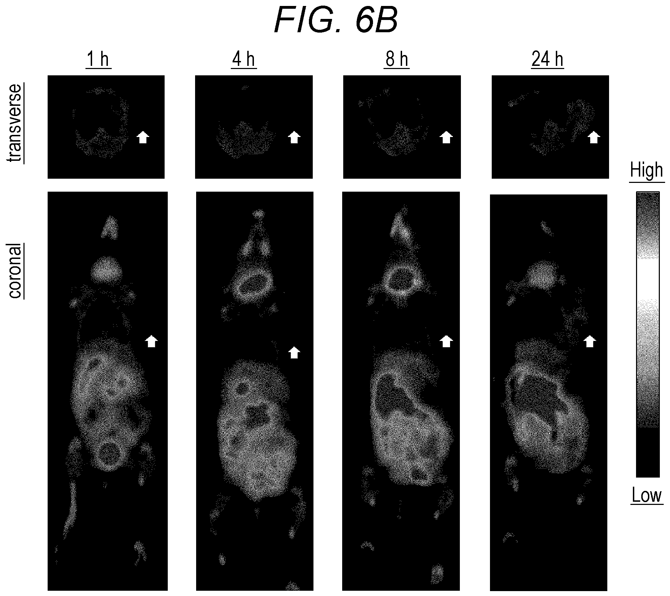

The collected data were reconstructed by a three-dimensional ordered-subset expectation maximization (3D-OSEM) method. The results of HT-29 cancer-bearing mouse are shown in Fig. 6A, and the results of MDA-MB-231 cancer-bearing mouse are shown in Fig. 6B.

In Figs. 6A and 6B, the arrows indicate the sites of tumor. After the lapse of 24 hours from the administration, as shown in Fig. 6A, the accumulation of radioactivity in the transplanted tumor was highly observed in the HT-29 cancer-bearing mouse, but as shown in Fig. 6B, the accumulation of [¹¹¹In]ITDA1 in the transplanted tumor was not observed in the MDA-MB-231 cancer-bearing mouse. Accordingly, it was confirmed that the HT-29 tumor can be selectively and clearly imaged by SPECT/CT by the [¹¹¹In]ITDA1.

### Example 17: Biodistribution of [¹¹¹In]ITDA3 in cancer-bearing mouse

HT-29 cells and MDA-MB-231 cells (5×10⁶ cells/mouse) were subcutaneously inoculated into the left shoulders of 5-week old male BALB/c nude mice, respectively. A saline solution (100 µL) in which [¹¹¹In]ITDA3 (40 kBq) had been dissolved was administered to each of the cancer-bearing mice (n = 5) via the tail vein. The HT-29 cancer-bearing mice were slaughtered after the lapse of 1, 4, and 24 hours from the administration, the MDA-MB-231 cancer-bearing mouse was slaughtered after the lapse of 24 hours from the administration, and the blood, spleen, pancreas, stomach, intestines, kidney, liver, heart, lungs, brain, HT-29 or MDA-MB-231-transplanted tumor, and muscle were recovered from each of the mice. The weight of each organ was measured, and the amount of radioactivity in each organ was measured by using a gamma counter. The accumulation of radioactivity in each organ except for stomach was expressed by % ID (injected dose)/g obtained by dividing the ratio of the amount of radioactivity of each organ to the administered amount of radioactivity by the organ weight. The stomach was expressed by % ID (injected dose). The results are shown in Table 5.

**Table 5**

| Organ | HT-29 Cancer-bearing mouse | | | MDA-MB-231 Cancer-bearing mouse |
|---|---|---|---|---|
| | 1 hour after administration | 4 hours after administration | 24 hours after administration | 24 hours after administration |
| Blood | 0.09 ± 0.01 | 0.06 ± 0.01 | 0.06 ± 0.01 | 0.06 ± 0.02 |
| Spleen | 0.13 ± 0.20 | 0.04 ± 0.03 | 0.08 ± 0.05 | 0.12 ± 0.02 |
| Pancreas | 14.89 ± 3.20 | 6.2 ± 0.78 | 3.85 ± 0.68 | 2.00 ± 0.54 |
| Stomach* | 1.80 ± 0.27 | 0.83 ± 0.11 | 0.48 ± 0.04 | 5.49 ± 0.55 |
| Intestines | 8.33 ± 0.44 | 7.2 ± 1.53 | 11.11 ± 0.85 | 6.66 ± 0.86 |
| Kidney | 95.81 ± 9.44 | 80.09 ± 5.11 | 70.19 ± 9.28 | 49.65 ± 5.04 |
| Liver | 8.42 ± 1.42 | 6.51 ± 1.50 | 4.92 ± 1.01 | 2.96 ± 0.64 |
| Heart | 6.91 ± 0.32 | 2.87 ± 0.36 | 2.00 ± 0.38 | 1.24 ± 0.19 |
| Lungs | 3.58 ± 0.86 | 2.24 ± 0.44 | 1.20 ± 0.15 | 0.93 ± 0.18 |
| Brain | 0.27 ± 0.05 | 0.26 ± 0.04 | 0.33 ± 0.07 | 0.24 ± 0.04 |
| HT-29 or MDA-MB-231 | 1.60 ± 0.16 | 1.71 ± 0.28 | 3.02 ± 0.37 | 0.72 ± 0.27 |
| Muscle | 2.47 ± 0.45 | 1.88 ± 0.13 | 1.63 ± 0.79 | 0.99 ± 0.26 |
| Tumor/blood | 18.89 ± 2.68 | 28.6 ± 3.24 | 52.06 ± 6.11 | 11.97 ± 6.08 |
| Tumor/muscl e | 0.66 ± 0.09 | 0.92 ± 0.15 | 2.08 ± 0.67 | 0.77 ± 0.32 |

| | | | | |
|---|---|---|---|---|
| * The unit is % ID only for the stomach, and % ID/g for the others. | | | | |

From the results in Table 5, the amount of tumor accumulation of [¹¹¹In]ITDA3 is lower than that of [¹¹¹In]ITDA1, but the amounts of accumulation in normal tissues (in particular, pancreas, stomach, intestines, and lungs) are small, and therefore, in a case where a trivalent radioactive metal element that emits alpha-rays or beta-minus rays was used as the radioactive metal, the reduction of side effects can be expected, and it was suggested that the [¹¹¹In]ITDA3 may be suitable as an internal radiotherapeutic agent.

### Example 18: In vitro CA-IX affinity and specificity of [ ¹¹¹In]ITDA1, [¹¹¹In] ITDA2, and [¹¹¹In] ITDA3 under 37°C condition

Example 18 was performed similarly as in Example 11 except that the cells were lysed in a constant temperature water bath at 37°C. The accumulation of radioactivity in each cell was expressed by % initial dose/mg protein obtained by dividing the ratio of the amount of radioactivity bound to each cell to the added amount of radioactivity by the amount of cell protein. The amounts of binding to HT-29 cells (% initial dose/mg protein) were as shown in Table 6. Further, the amounts of binding to MDA-MB-231 cells (% initial dose/mg protein) were as shown in Table 7.

**Table 6**

| | [¹¹¹In] ITDA1 | [¹¹¹In] ITDA 2 | [¹¹¹In] ITDA 3 |
|---|---|---|---|
| 21% Oxygen AZ(-) | 118.1 ± 21.4 | 48.3 ± 5.9 | 47.2 ± 6.8 |
| 21% Oxygen AZ(+) | 0.9 ± 0.2 | 7.6 ± 2.3 | 0.3 ± 0.0 |
| 1% Oxygen AZ(-) | 198.9 ± 5.7 | 109.2 ± 6.7 | 93.4 ± 16.0 |
| 1% Oxygen AZ(+) | 2.2 ± 0.5 | 12.1 ± 3.7 | 1.0 ± 0.1 |

**Table 7**

| | [¹¹¹In] ITDA1 | [¹¹¹In] ITDA 2 | [¹¹¹In] ITDA 3 |
|---|---|---|---|
| 21% Oxygen AZ(-) | 1.4 ± 0.3 | 2.5 ± 0.5 | 0.5 ± 0.1 |
| 21% Oxygen AZ(+) | 0.1 ± 0.0 | 1.4 ± 0.2 | 0.2 ± 0.0 |
| 1% Oxygen AZ(-) | 6.5 ± 0.8 | 2.9 ± 0.4 | 2.0 ± 1.1 |
| 1% Oxygen AZ(+) | 0.1 ± 0.0 | 0.7 ± 0.1 | 0.1 ± 0.0 |

As shown in Table 6, the inhibition was observed in the presence of acetazolamide under both normoxic and hypoxic conditions in the CA-IX-positive HT-29 cells, but the inhibition was not observed in the absence of the competitive compound. On the other hand, as shown in Table 7, the competitive inhibition was not observed regardless of the presence or absence of acetazolamide under both normoxic and hypoxic conditions in the CA-IX-negative MDA-MB-231 cells. From the above results, even in a case where the cells were lysed at 37°C, similarly to the results of room temperature conditions (Example 11), it was indicated that [¹¹¹In]ITDA1, [¹¹¹In]ITDA2, and [¹¹¹In]ITDA3 selectively bind to the CA-IX.

### Example 19: Biodistribution of [¹¹¹In]ITDA2 in cancer-bearing mouse

HT-29 cells (5×10⁶ cells/mouse) and MDA-MB-231 cells (1×10⁷ cells/mouse) were subcutaneously inoculated into the left shoulders of 5-week old male BALB/c nude mice, respectively, and the mice were used when the tumor diameter reached 6 to 10 mm. A saline solution (100 µL) in which [¹¹¹In]ITDA2 (40 kBq) had been dissolved was administered to each of the HT-29 tumor or MDA-MB-231 tumor cancer-bearing mice (n = 5) via the tail vein. The HT-29 tumor cancer-bearing mice were slaughtered after the lapse of 1, 4, 8, and 24 hours from the administration, the MDA-MB-231 tumor cancer-bearing mouse was slaughtered after the lapse of 24 hours from the administration, and the blood, spleen, pancreas, stomach, intestines, kidney, liver, heart, lungs, brain, tumor, and muscle were recovered from each of the mice. The weight of each organ was measured, and the amount of radioactivity in each organ was measured by using a gamma counter. The accumulation of radioactivity in each organ except for stomach was expressed by % injected dose/g organ obtained by dividing the ratio of the amount of radioactivity of each organ to the administered amount of radioactivity by the organ weight. The stomach was expressed by % injected dose. The results are shown in Tables 8 and 9.

**Table 8: Biokinetics in HT-29 tumor cancer-bearing mouse of [¹¹¹In]ITDA2 (% injected dose/g organ)**

| Organ/tissue | 1 hour | 4 hours | 8 hours | 24 hours |
|---|---|---|---|---|
| Blood | 0.27 ± 0.03 | 0.18 ± 0.01 | 0.20 ± 0.01 | 0.26 ± 0.02 |
| Spleen | 0.40 ± 0.27 | 0.13 ± 0.10 | 0.32 ± 0.20 | 0.27 ± 0.13 |
| Pancreas | 20.01 ± 6.77 | 20.88 ± 2.49 | 13.15 ± 4.17 | 7.23 ± 1.40 |
| Stomach* | 3.99 ± 0.49 | 5.09 ± 0.66 | 6.78 ± 0.95 | 9.33 ± 0.72 |
| Intestines | 11.24 ± 1.54 | 15.32 ± 1.51 | 15.40 ± 0.94 | 11.14 ± 2.06 |
| Kidney | 137 ± 11.1 | 143 ± 12.0 | 110 ± 13.7 | 76.4 ± 17.2 |
| Liver | 4.11 ± 0.54 | 4.87 ± 1.08 | 4.58 ± 0.76 | 3.50 ± 0.81 |
| Heart | 5.64 ± 1.01 | 2.81 ± 0.39 | 1.89 ± 0.35 | 1.58 ± 0.42 |
| Lungs | 6.10 ± 0.97 | 4.25 ± 0.76 | 2.87 ± 1.08 | 2.15 ± 0.49 |
| Brain | 0.30 ± 0.04 | 0.30 ± 0.04 | 0.35 ± 0.05 | 0.37 ± 0.09 |
| Tumor | 2.11 ± 0.32 | 2.47 ± 0.30 | 3.10 ± 0.35 | 5.93 ± 0.88 |
| Muscle | 2.05 ± 0.61 | 2.00 ± 0.21 | 1.39 ± 0.20 | 1.00 ± 0.25 |
| Tumor/blood | 7.78 ± 0.95 | 13.85 ± 2.45 | 15.31 ± 1.69 | 22.63 ± 3.31 |
| Tumor/muscle | 1.12 ± 0.45 | 1.24 ± 0.19 | 2.28 ± 0.39 | 6.18 ± 1.45 |

| | | | | |
|---|---|---|---|---|
| *Expressed in % injected dose | | | | |

**Table 9: Biokinetics in MDA-MB-231 tumor cancer-bearing mouse of [¹¹¹In]ITDA2 (% injected dose/g organ)**

| Organ/tissue | 24 hours |
|---|---|
| Blood | 0.29 ± 0.04 |
| Spleen | 0.27 ± 0.07 |
| Pancreas | 3.37 ± 0.28 |
| Stomach* | 8.43 ± 1.08 |
| Intestines | 9.15 ± 1.04 |
| Kidney | 59.8 ± 4.04 |
| Liver | 2.77 ± 0.45 |
| Heart | 1.13 ± 0.19 |
| Lungs | 1.70 ± 0.57 |
| Brain | 0.34 ± 0.07 |
| Tumor | 1.56 ± 0.17 |
| Muscle | 0.64 ± 0.06 |
| Tumor/blood | 5.51 ± 0.78 |
| Tumor/muscle | 2.46 ± 0.39 |

| | |
|---|---|
| *Expressed in % injected dose | |

From Tables 8 and 9, the tumor/muscle ratio of ITDA2 was almost the same value as that of ITDA1, but the tumor accumulation and the tumor/blood ratio of ITDA2 were lower values as compared with those of ITDA1. This is considered to correlate with the results of Examples 11 and 18.

The above embodiments include the following technical ideas.
(1) A radiolabeled compound represented by the following formula (1) or a salt thereof: 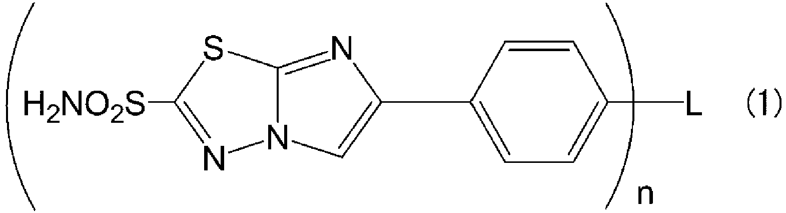 wherein n is an integer of 1 to 4, and L represents a radionuclide or a mono- to tetravalent group containing a radionuclide.
(2) The radiolabeled compound or a salt thereof described in (1), wherein n is 1, and L represents a radioactive halogen atom, an alkyl group having 1 to 10 carbon atoms which is replaced at a terminal thereof with a radioactive halogen atom, or a polyethylene glycol group having 1 to 10 carbon atoms which is replaced at a terminal thereof with a radioactive halogen atom.
(3) The radiolabeled compound or a salt thereof described in (2), wherein the radioactive halogen atom is a radioactive iodine atom.
(4) The radiolabeled compound or a salt thereof described in (1), wherein L represents a mono- to tetravalent ligand carrying a radioactive metal.
(5) The radiolabeled compound or a salt thereof described in (4), wherein the ligand is DOTA which may have a carboxyl terminus that is amidated.
(6) The radiolabeled compound or a salt thereof described in (4) or (5), wherein the radioactive metal is ¹¹¹In, ⁹⁰Y, ⁶⁷Ga, ⁶⁸Ga, ¹⁷⁷Lu, ^{99m}Tc, ⁶⁴Cu, ¹⁵³Gd, ²¹³Bi, or ²²⁵Ac.
(7) The radiolabeled compound or a salt thereof described in any one of (4) to (6), represented by the following formula (1-1) or (1-2): 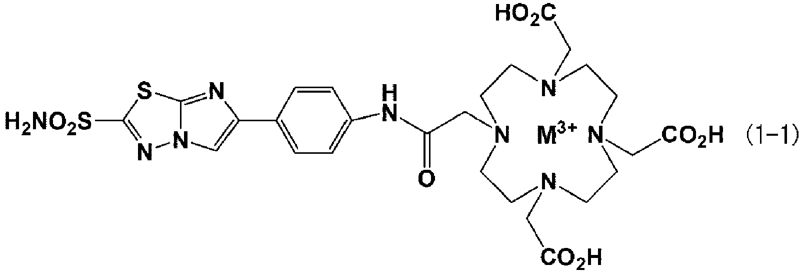 wherein M³⁺ represents a trivalent radioactive metal element, or 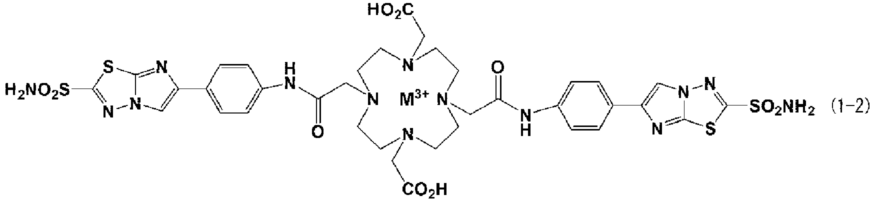 wherein M³⁺ represents a trivalent radioactive metal element.
(8) A radiopharmaceutical, comprising the radiolabeled compound or a salt thereof described in any one of (1) to (8) as an active component.
(9) The radiopharmaceutical described in (8), which is an imaging agent for a tumor.
(10) The radiopharmaceutical described in (8), which is an internal radiotherapeutic agent for a tumor.
(11) A compound represented by the following formula (2) or a salt thereof: 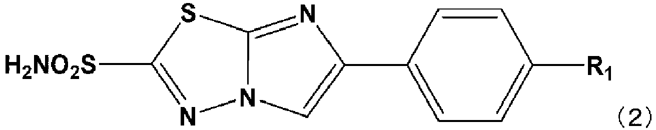 wherein R₁ represents a non-radioactive halogen atom, a nitro group, a trialkyl ammonium group, a dialkyl sulfonium group, a trialkyl stannyl group, a triphenyl stannyl group, a trialkyl silyl group, a triphenyl silyl group, an alkyl group having 1 to 10 carbon atoms which is replaced at a terminal thereof with a sulfonyloxy group, or a polyethylene glycol group having 1 to 10 carbon atoms which is replaced at a terminal thereof with a sulfonyloxy group.
(12) A method for producing a radioactive halogen-labeled compound represented by the following formula (3) or a salt thereof: 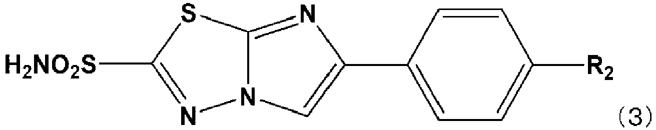 wherein R₂ represents a radioactive halogen atom, an alkyl group having 1 to 10 carbon atoms which is replaced at a terminal thereof with a radioactive halogen atom, or a polyethylene glycol group having 1 to 10 carbon atoms which is replaced at a terminal thereof with a radioactive halogen atom,
   from a compound represented by the following formula (2) or a salt thereof: 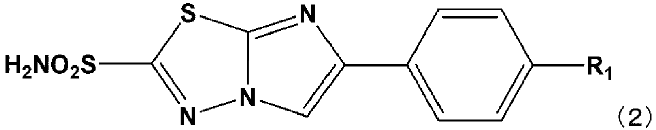 wherein R₁ represents a non-radioactive halogen atom, a nitro group, a trialkyl ammonium group, a dialkyl sulfonium group, a trialkyl stannyl group, a triphenyl stannyl group, a trialkyl silyl group, a triphenyl silyl group, an alkyl group having 1 to 10 carbon atoms which is replaced at a terminal thereof with a sulfonyloxy group, or a polyethylene glycol group having 1 to 10 carbon atoms which is replaced at a terminal thereof with a sulfonyloxy group,
   by a radioactive halogenation reaction.
(13) A compound represented by the following formula (2-1) or (2-2) or a salt thereof. 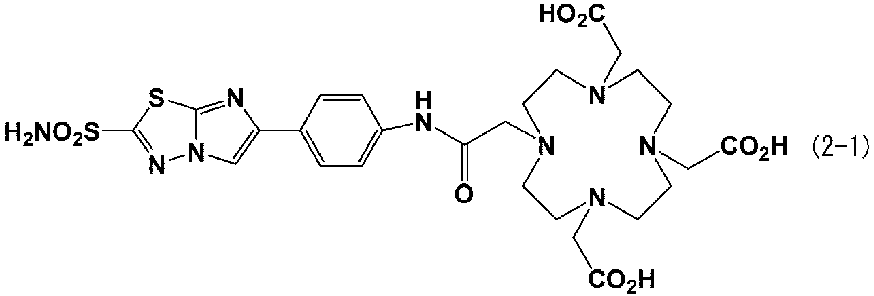 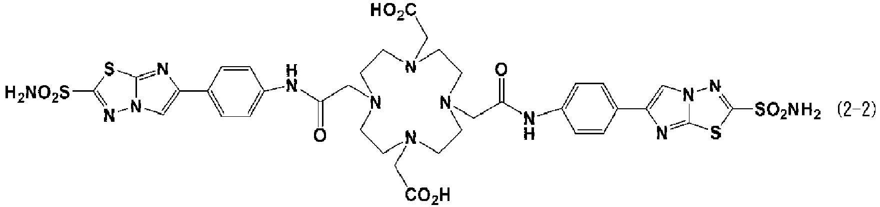
(14) A complex of the compound or a salt thereof described in (13) with a radioactive metal.
(15) A method for producing a radioactive metal complex, comprising mixing the compound or a salt thereof described in (13) with a radioactive metal to obtain the complex described in (14).
(16) A kit for preparing the complex described in (14), comprising the compound or a salt thereof described in (13).
(17) Use of the compound or a salt thereof described in any one of (1) to (7), (11), and (13) in production of a medicament.

This application claims the priority based on Japanese Patent Application No. 2018-162085 filed on August 30, 2018, Japanese Patent Application No. 2018-208322 filed on November 5, 2018, and Japanese Patent Application No. 2019-37734 filed on March 1, 2019, and the disclosure of each of which is incorporated herein by reference in its entirety.

## Claims

1. A radiolabeled compound represented by the following formula (1) or a salt thereof: 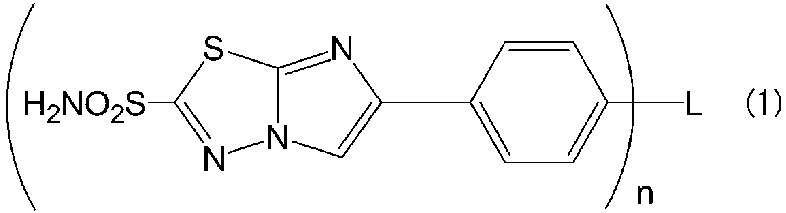 wherein n is an integer of 1 to 4, and L represents a radionuclide or a mono- to tetravalent group containing a radionuclide.

2. The radiolabeled compound or a salt thereof according to claim 1, wherein
n is 1, and L represents a radioactive halogen atom, an alkyl group having 1 to 10 carbon atoms which is replaced at a terminal thereof with a radioactive halogen atom, or a polyethylene glycol group having 1 to 10 carbon atoms which is replaced at a terminal thereof with a radioactive halogen atom.

3. The radiolabeled compound or a salt thereof according to claim 2, wherein
the radioactive halogen atom is a radioactive iodine atom.

4. The radiolabeled compound or a salt thereof according to claim 1, wherein
L represents a mono- to tetravalent ligand carrying a radioactive metal.

5. The radiolabeled compound or a salt thereof according to claim 4, wherein
the ligand is DOTA which may have a carboxyl terminus that is amidated.

6. The radiolabeled compound or a salt thereof according to claim 4, wherein
L represents a ligand represented by the following formula (1-3) or formula (1-4) in which a radioactive metal is carried: 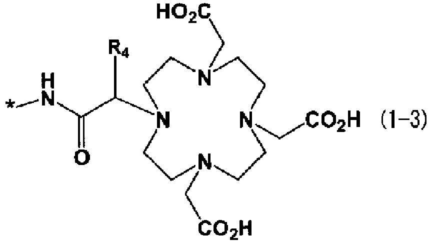 wherein R₄ represents H or CO₂H, and a pentagram asterisk represents a binding site, or 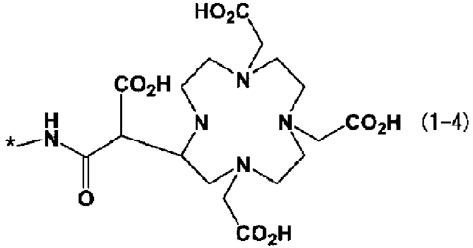 wherein a pentagram asterisk represents a binding site,
and n is 1.

7. The radiolabeled compound or a salt thereof according to any one of claims 4 to 6, wherein
the radioactive metal is ¹¹¹In, ⁹⁰Y, ⁶⁷Ga, ⁶⁸Ga, ¹⁷⁷Lu, ^{99m}Tc, ⁶⁴Cu, ¹⁵³Gd, ²¹³Bi, or ²²⁵Ac.

8. The radiolabeled compound or a salt thereof according to any one of claims 4 to 7, represented by the following formula (1-1) or (1-2): 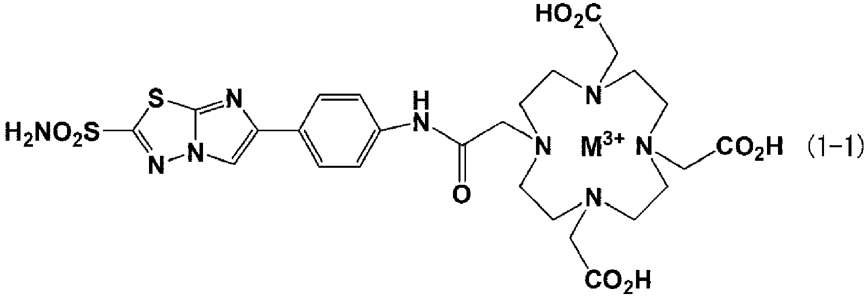 wherein M³⁺ represents a trivalent radioactive metal element, or 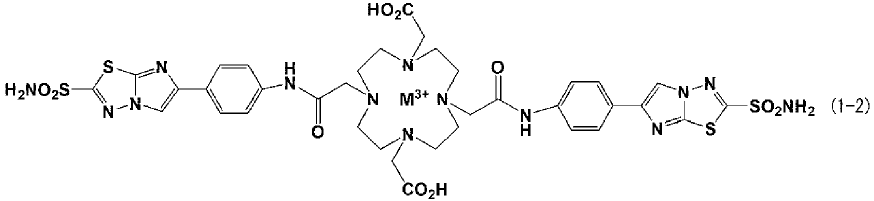 wherein M³⁺ represents a trivalent radioactive metal element.

9. A radiopharmaceutical comprising
the radiolabeled compound or a salt thereof according to any one of claims 1 to 8 as an active component.

10. The radiopharmaceutical according to claim 9, which is an imaging agent for a tumor.

11. The radiopharmaceutical according to claim 9, which is an internal radiotherapeutic agent for a tumor.

12. A compound represented by the following formula (2) or a salt thereof: 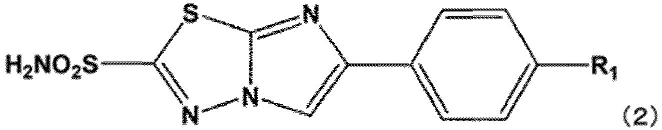 wherein R₁ represents a non-radioactive halogen atom, a nitro group, a trialkyl ammonium group, a dialkyl sulfonium group, a trialkyl stannyl group, a triphenyl stannyl group, a trialkyl silyl group, a triphenyl silyl group, an alkyl group having 1 to 10 carbon atoms which is replaced at a terminal thereof with a sulfonyloxy group, or a polyethylene glycol group having 1 to 10 carbon atoms which is replaced at a terminal thereof with a sulfonyloxy group.

13. A method for producing a radioactive halogen-labeled compound represented by the following formula (3) or a salt thereof: 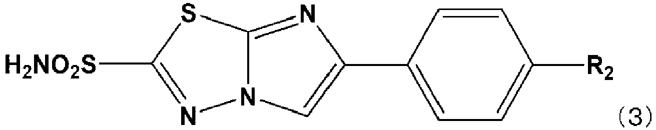 wherein R₂ represents a radioactive halogen atom, an alkyl group having 1 to 10 carbon atoms which is replaced at a terminal thereof with a radioactive halogen atom, or a polyethylene glycol group having 1 to 10 carbon atoms which is replaced at a terminal thereof with a radioactive halogen atom,
which comprises a radioactive halogenation reaction of a compound represented by the following formula (2) or a salt thereof: 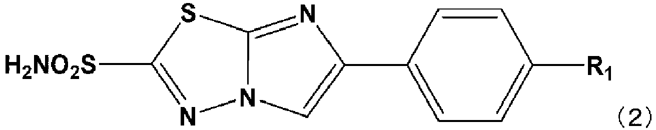 wherein R₁ represents a non-radioactive halogen atom, a nitro group, a trialkyl ammonium group, a dialkyl sulfonium group, a trialkyl stannyl group, a triphenyl stannyl group, a trialkyl silyl group, a triphenyl silyl group, an alkyl group having 1 to 10 carbon atoms which is replaced at a terminal thereof with a sulfonyloxy group, or a polyethylene glycol group having 1 to 10 carbon atoms which is replaced at a terminal thereof with a sulfonyloxy group.

14. A compound represented by the following formula (2-1), (2-2), (3-1), or (3-2), or a salt thereof: 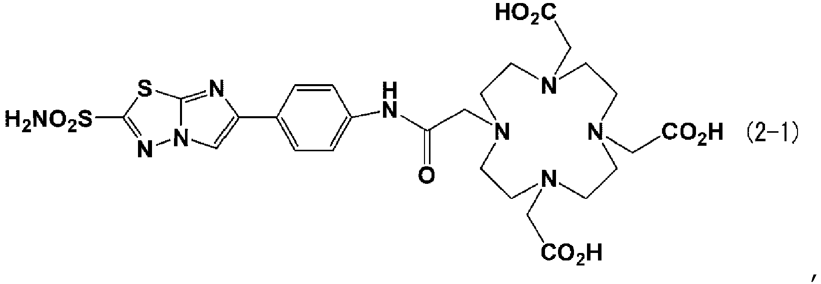 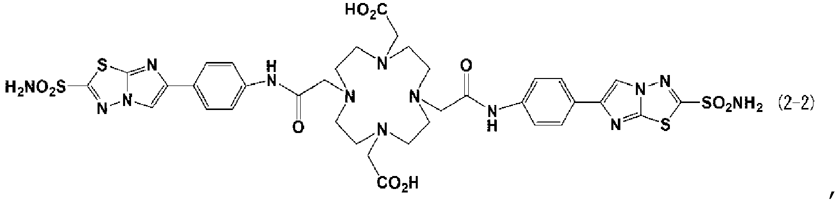 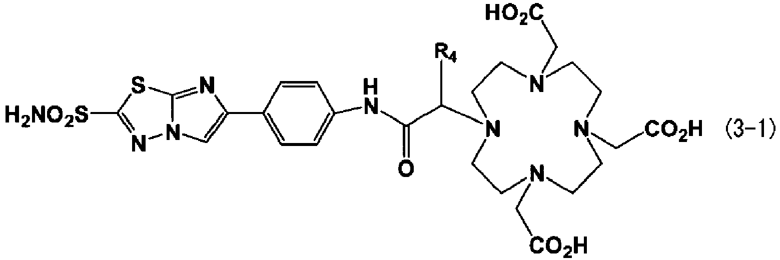 wherein R₄ represents H or CO₂H, or

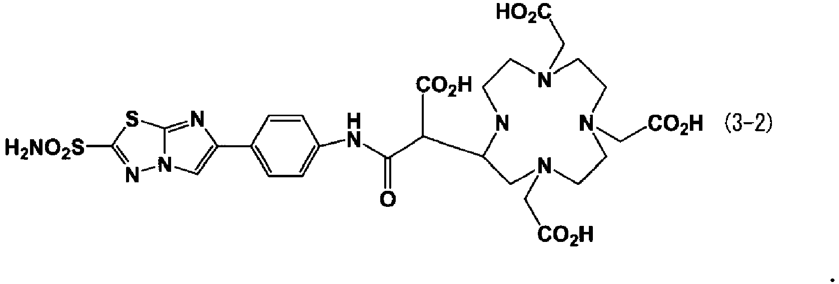

15. A complex comprising the compound or a salt thereof according to claim 14 with a radioactive metal.

16. A method for producing a radioactive metal complex, comprising
mixing the compound or a salt thereof according to claim 14 with a radioactive metal to obtain the complex according to claim 15.

17. A kit for preparing the complex according to claim 15, comprising
the compound or a salt thereof according to claim 14.

18. Use of the compound or a salt thereof according to any one of claims 1 to 7, 12, and 14 in production of a medicament.
